# EUROPEAN PATENT APPLICATION

(11) **EP 4 624 494 A1**
(43) Date of publication of application: **01.10.2025**
(21) Application number: 24305501.9
(22) Date of filing: 29.03.2024
(51) Int. Cl.: C07K 16/32, A61K 39/00, C07K 14/725, A61P 35/00

(54) **HER2 SINGLE DOMAIN ANTIBODY AND USES THEREOF**

(71) Applicant: Institut Curie, 75005 Paris (FR); Centre National de la Recherche Scientifique, 75016 Paris (FR)
(72) Inventor:
(74) Representative: Plasseraud IP

(57) **Abstract**

The invention relates to a fully humanized anti-HER2 single domain antibody (sdAb) and variants thereof. The present invention further relates to nucleic acids, vectors, host cells, immune cells, functionalized drug nanocarriers comprising said sdAb, and compositions comprising thereof. The invention also relates to therapeutic and diagnostic uses thereof and to methods and pharmaceutical compositions for the treatment of cancer. The invention also relates to chimeric antigen receptors including said humanized HER2 sdAb in their antigen binding domain and their use in cancer cell therapy.

## Description

### FIELD OF THE INVENTION:

The invention relates to anti-HER2 single domain antibody (sdAb) and variants thereof. The invention also relates to therapeutic and diagnostic uses thereof and to methods and pharmaceutical compositions for the treatment of cancer. The invention also relates to chimeric antigen receptors including said humanized HER2 sdAb in their antigen binding domain and their use in cancer cell therapy.

### BACKGROUND OF THE INVENTION:

HER2, also known as ERBB2 (human), proto-oncogene Neu, or even CD340 (cluster of differentiation 340), is a member of the human epidermal growth factor receptor (HER/EGFR/ERBB) family. The overexpression of HER2 is correlated with cell proliferation and tumorigenesis and occurs in various cancers such as approximately 20% to 30% of breast cancers , about 7% to 34% of gastric cancers and in about 30% of salivary duct carcinomas. HER 2 is further expressed in a variety of other human cancers, such as ovarian, adenocarcinoma of the lung, and aggressive forms of uterine cancer (Burstein HJ. The distinctive nature of HER2-positive breast cancers, N Engl J Med. 2005;353:1652-1654; Ruschoff J et al., HER2 testing in gastric cancer: a practical approach. Mod Pathol. 2012;25:637-650; Meza-Junco J, Au HJ, Sawyer MB. Critical appraisal of trastuzumab in treatment of advanced stomach cancer, Cancer Manag Res. 2011;3:57-64; Chiosea SI, et al., Molecular characterization of apocrine salivary duct carcinoma. Am J Surg Pathol. 2015;39:744-752). Her2-positive tumors are generally correlated aggressive cancer forms and a poorer prognosis. Several therapeutic methods have been developed to block Her2 activity to suppress tumor growth, notably monoclonal antibodies (mAbs) such as trastuzumab (Santin AD et al., Trastuzumab treatment in patients with advanced or recurrent endometrial carcinoma overexpressing HER2/neu. Int J Gynecol Obstet. 2008;102:128-131; Vasconcellos FA et al., Generation and characterization of new HER2 monoclonal antibodies. Acta Histochem. 2013;115:240-244). Although treatment with trastuzumab and other HER2-directed therapies are associated with significant efficacy, only patients with the highest levels of HER2 expression, representing approximately 20% of breast cancer patients, have the potential to respond. Moreover, many patients expressing high levels of HER2 progress or relapse despite receiving the best HER2-directed treatments, and thus require novel treatment approaches. For some patients also these therapeutics show significant clinical benefits, their efficacy remains variable and modest, for example, with no benefit against Her2-positive head and neck cancer (Pollock NI, Grandis JR., HER2 as a therapeutic target in head and neck squamous cell carcinoma. Clin Cancer Res. 2015;21:526-533; Wu X, Chen S, Lin L, et al. A Single Domain-Based Anti-Her2 Antibody Has Potent Antitumor Activities. Transl Oncol. 2018;11(2):366-373). Thus, it is necessary to develop new therapeutic avenues to improve the current Her2-targeting therapy.

Chimeric antigen receptor (CAR) technology has revolutionized the field of immunotherapy, demonstrating highly efficient in the fight against hematologic malignancies. These results leaded to Food and Drug Administration (FDA) and European Medicines Agency (EMA) approval of two CAR-T cell therapies using an scFv-CD19 against malignant B cells, the Klymriah and Yescarta. Unfortunately, the breakthrough with CAR-T cell therapy in the treatment of hematologic malignancies is still not well replicated in solid tumors (Y. Guo, Y et al., Chimeric antigen receptor-modified T cells for solid tumors: challenges and prospects, J Immunol Res, 2016; J. Li et al., Chimeric antigen receptor T cell (CAR-T) immunotherapy for solid tumors: lessons learned and strategies for moving forward; J Hematol Oncol, 11 (2018), p. 22). Until now, no CAR-T cells against solid tumor has shown such efficiency. Furthermore, scFvs, which are mostly used in the design of chimeric antigen receptors exhibit a number of characteristics that may negatively impact on the therapeutic efficacy of CAR-Ts. Indeed, scFv are notably characterized by poor expression and stability and are prone to unfolding and aggregation.

Therefore, it remains crucial the development and optimization of CAR-T cells against solid tumor but also with limited side effects that could compromise the patient's life. CARs are usually composed by an antibody-derived fragment, usually a single chain variable fragment (scFv), fused to a transmembrane domain and co-stimulatory motives required for immune cells survival, persistence and effector activity.

Thus, there remains a constant need to improve and diversify current therapeutic tools in oncology to cover not only the diversity of patient profiles but also the significant variability of tumours. This is particularly critical for aggressive tumours related to HER2 overexpression.

### SUMMARY OF THE INVENTION:

The invention relates to a fully humanized anti-HER2 single domain antibody (sdAb) and variants thereof. The present invention further relates to nucleic acids, vectors, host cells, immune cells, functionalized drug nanocarriers comprising said sdAb, and compositions comprising thereof. The invention also relates to therapeutic and diagnostic uses thereof and to methods and pharmaceutical compositions for the treatment of cancer. The invention also relates to chimeric antigen receptors including said humanized HER2 sdAb in their antigen binding domain and their use in cancer cell therapy. In particular, the invention is defined by the claims.

### DETAILED DESCRIPTION OF THE INVENTION:

The inventors selected from their humanized sdAb libraries a fully humanized anti-HER2 single domain antibody (sdAb) that selectively detect the surface of breast tumor cells. The inventors demonstrated that the anti-HER2 sdAb of the invention shown in FACS experiment specific recognition on SKBR3 cells which overexpresses the HER2 protein on its surface and not on MCF10A cells derived from non-cancerous and HER2-negative mammary gland cells. The anti-HER2 sdAb was also validated by ELISA on Her2 recombinant ectodomain fused to a Rabbit Fc domain. The inventors also validated the anti-HER2 sdAb of the invention for use in CAR-T format. The anti-Her2 sdAb of the invention showed specific activation that can be detected by FACS. Again, CAR including the anti-Her2 sdAb of the invention shows a better T cell activation than CAR including scFv and anti-Her2 VHH. The invention also performed *in vivo* experiments using the anti-HER2 sdAb CAR-T of the invention and a comparison with the scFv CAR-T of reference in a grafted Her2 tumors mouse model. The inventors also compare *in vivo* the activity of the sdAb of the invention in preclinical models with the CAR-T derived from Trastuzumab. This study allows to obtain comparison data with a reference treatment and to demonstrate the benefits of the technology and the potential of the CAR-T product armed with sdAb of the invention. The inventors demonstrated that the CAR-T cells armed with the anti-HER2 sdAb of the invention was expressed on the surface of T lymphocytes and achieved a high percentage of killed tumor cells. Mice injected with anti-Her2 CAR-T cells saw their tumors disappear demonstrating the efficacy of the sdAb of the invention in CAR-T. The result of the present invention is the first proof of concept for the use of sdAbs *in vivo* in CAR-T format with an anti-tumor effect against the Her2 target.

Accordingly, the invention relates to a fully humanized anti-HER2 single domain antibody (sdAb) and variants thereof. The present invention further relates to nucleic acids, vectors, host cells, immune cells, functionalized drug nanocarriers comprising said sdAb, and compositions comprising thereof. The invention also relates to therapeutic and diagnostic uses thereof and to methods and pharmaceutical compositions for the treatment of cancer. The invention also relates to chimeric antigen receptors including said humanized HER2 sdAb in their antigen binding domain and their use in cancer cell therapy.

### Definitions:

The terminology used herein is for the purpose of describing particular embodiments only and is not intended to be exhaustive. It must be noted that, as used in the specification and the appended claims, the singular forms "a," "an" and "the" include plural referents unless the context clearly dictates otherwise.

The term "comprising" as used herein is synonymous with "including" or "containing" and is inclusive or open-ended and does not exclude additional, uncited members, elements or method steps.

Unless specifically stated or obvious from context, as used herein, the term "about" is to be understood as within a range of normal tolerance in the art, for example within 2 standard deviations of the mean. About can be understood as within 20%, 15%, 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

As used herein, the term "isolated" refers to a substance or entity that has been (1) separated from at least some of the components with which it was associated when initially produced (whether in nature or in an experimental setting), and (2) produced, prepared, and/or manufactured by the hand of man. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91 %, about 92%, about 93%, about 94%, about 95%, about 96%, about 97%, about 98%, about 99%, or more than about 99% pure. As used herein, a substance is "pure" if it is substantially free of other components.

The "isolated" products of the present disclosure, including isolated nucleic acids, proteins, polypeptides, and antibodies are not products of nature (i.e., "non-naturally occurring"). Rather, the "isolated" nucleic acids, proteins, polypeptides, and antibodies of the present disclosure are "man-made" products. The "isolated" products of the present disclosure can be "markedly different" or "significantly different" from products of nature. By way of a non-limiting example, the isolated nucleic acids may be purified, recombinant, synthetic, labeled, and/or attached to a solid substrate. Such nucleic acids can be markedly different or significantly different than nucleic acids that occur in nature. By way of further non-limiting example, the "isolated" proteins, polypeptides, and antibodies of the present disclosure may be purified, recombinant, synthetic, labeled, and/or attached to a solid substrate. Such proteins, polypeptides, and antibodies can be markedly different or significantly different from proteins, polypeptides, and antibodies that occur in nature.

The term "polynucleotide", "nucleic acid molecule", "nucleic acid", or "nucleic acid sequence" refers to a polymeric form of nucleotides of at least 10 bases in length. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation. The nucleic acid (also referred to as polynucleotides) may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. Other modifications can include, for example, analogs in which the ribose ring contains a bridging moiety or other structure such as the modifications found in "locked" nucleic acids.

A "synthetic" RNA, DNA or a mixed polymer is one created outside of a cell, for example one synthesized chemically.

The term "nucleic acid fragment" as used herein refers to a nucleic acid sequence that has a deletion, e.g., a 5'-terminal or 3'-terminal deletion compared to a full-length reference nucleotide sequence. In an embodiment, the nucleic acid fragment is a contiguous sequence in which the nucleotide sequence of the fragment is identical to the corresponding positions in the naturally-occurring sequence. In some embodiments, fragments are at least 10, 15, 20, or 25 nucleotides long, or at least 20, 30, 40, 50, 60, 70, 80, 90, 100, 1 10, 120, 130, 140, or 150 nucleotides long. In some embodiments a fragment of a nucleic acid sequence is a fragment of an open reading frame sequence. In some embodiments such a fragment encodes a polypeptide fragment (as defined herein) of the protein encoded by the open reading frame nucleotide sequence.

The nucleic acid can be purified. Preferably, the purified nucleic acid is more than 50%, 75%, 85%, 90%, 95%, 97%, 98%, or 99% pure. Within the context of the present disclosure, a purified nucleic acid that is at least 50% pure means a purified nucleic acid sample containing less than 50% other nucleic acids. For example, a sample of a plasmid can be at least 99% pure if it contains less than 1 % contaminating bacterial DNA.

The term "operably linked" in the context of nucleic acids refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof. Further, a polypeptide may comprise a number of different domains each of which having one or more distinct activities. For the avoidance of doubt, a "polypeptide" may be any length greater two amino acids.

The term "peptide" as used herein refers to a short polypeptide, e.g., one that typically contains less than about 50 amino acids and more typically less than about 30 amino acids. The term as used herein encompasses analogs and mimetics that mimic structural and thus biological function.

The term "isolated protein" or "isolated polypeptide" is a protein or polypeptide that by virtue of its origin or source of derivation (1) is not associated with naturally associated components that accompany it in its native state, (2) exists in a purity not found in nature, where purity can be adjudged with respect to the presence of other cellular material (e.g., is free of other proteins from the same species) (3) is expressed by a cell from a different species, or (4) does not occur in nature (e.g., it is a fragment of a polypeptide found in nature or it includes amino acid analogs or derivatives not found in nature or linkages other than standard peptide bonds). Thus, a polypeptide that is chemically synthesized or synthesized in a cellular system different from the cell from which it naturally originates will be "isolated" from its naturally associated components. A polypeptide or protein may also be rendered substantially free of naturally associated components by isolation, using protein purification techniques well known in the art. As thus defined, "isolated" does not necessarily require that the protein, polypeptide, peptide or oligopeptide so described has been physically removed from a cell in which it was synthesized.

The protein or polypeptide can be purified. Preferably, the purified protein or polypeptide is more than 50%, 75%, 85%, 90%, 95%, 97%, 98%, or 99% pure. Within the context of the present disclosure, a purified protein that is more than 50% (etc.) pure means a purified protein sample containing less than 50% (etc.) other proteins. For example, a sample of a protein comprising can be 99% pure if it contains less than 1 % contaminating host cell proteins.

The term "polypeptide fragment" as used herein refers to a polypeptide that has a deletion, e.g., an amino-terminal and/or carboxy-terminal deletion compared to a full-length polypeptide, such as a naturally occurring protein. In an embodiment, the polypeptide fragment is a contiguous sequence in which the amino acid sequence of the fragment is identical to the corresponding positions in the naturally-occurring sequence. Fragments typically are at least 5, 6, 7, 8, 9 or 10 amino acids long, or at least 12, 14, 16 or 18 amino acids long, or at least 20 amino acids long, or at least 25, 30, 35, 40 or 45, amino acids, or at least 50 or 60 amino acids long, or at least 70 amino acids long, or at least 100 amino acids long.

The terms "percent identical" or "percent identity," in the context of two or more nucleic acids or polypeptide sequences, refers to the extent to which two or more sequences or subsequences that are the same. Two sequences are "identical" if they have the same sequence of amino acids or nucleotides over the region being compared. Two sequences are "substantially identical" if two sequences have a specified percentage of amino acid residues or nucleotides that are the same (i.e., 60% identity, optionally 65%, 70%, 75%, 80%, 85%, 90%, 91% 92%, 93%, 94%, 95%, 96%, 97%, 98% or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 30 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman, Adv. Appl. Math. 2:482c (1970), by the homology alignment algorithm of Needleman and Wunsch, J. Mol. Biol. 48:443 (1970), by the search for similarity method of Pearson and Lipman, Proc. Natl. Acad. Sci. USA 85 :2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g. , Brent et al., Current Protocols in Molecular Biology, 2003).

Two examples of algorithms that are suitable for determining percent sequence identity and sequence similarity are the BLAST and BLAST 2.0 algorithms, which are described in Altschul et al., Nuc. Acids Res. 25:3389-3402, 1977; and Altschul et al., J. Mol. Biol. 215:403-410, 1990, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) or 10, M=5, N=-4 and a comparison of both strands. For amino acid sequences, the BLASTP program uses as defaults a word length of 3, and expectation (E) of 10, and the BLOSUM62 scoring matrix (see Henikoff and Henikoff, (1989) Proc. Natl. Acad. Sci. USA 89: 10915) alignments (B) of 50, expectation (E) of 10, M=5, N=-4, and a comparison of both strands. The BLAST algorithm also performs a statistical analysis of the similarity between two sequences (see, e.g., Karlin and Altschul, Proc. Natl. Acad. Sci. USA 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller, Comput. Appl. Biosci. 4: 11 -17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch, J. Mol. Biol. 48:444-453, 1970) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1 , 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

As used herein a "functional variant" or a given protein includes the wild-type version of said protein, a variant protein belonging to the same family, an homolog protein, or a truncated version, which preserves the functionality of the given protein. Typically the functional variant exhibit at least 70%, 75%, 80%, 85%,90%, 95%, 97%, 98%, or 99% amino acid identity with the given protein.

As used herein, the term "mammal" refers to any member of the taxonomic class mammalian, including placental mammals and marsupial mammals. Thus, "mammal" includes humans, primates, livestock, and laboratory mammals. Exemplary mammals include a rodent, a mouse, a rat, a rabbit, a dog, a cat, a sheep, a horse, a goat, a llama, cattle, a primate, a pig, and any other mammal. In some embodiments, the mammal is at least one of a transgenic mammal, a genetically-engineered mammal, and a cloned mammal.

According to the present disclosure, the term "disease" refers to any pathological state, including cancer diseases, in particular those forms of cancer diseases described herein.

The term "normal" refers to the healthy state or the conditions in a healthy subject or tissue, i.e., non-pathological conditions, wherein "healthy" preferably means non-cancerous.

The term "malignancy" refers to a non-benign tumor or a cancer. As used herein, the term "cancer" includes a malignancy characterized by deregulated or uncontrolled cell growth.

The term "cancer" includes primary malignant tumors (e.g., those whose cells have not migrated to sites in the subject's body other than the site of the original tumor) and secondary malignant tumors (e.g., those arising from metastasis, the migration of tumor cells to secondary sites that are different from the site of the original tumor).

Cancers are classified by the type of cell that resembles the tumor and, therefore, the tissue presumed to be the origin of the tumor. These are the histology and the location, respectively.

The term "cancer" according to the disclosure comprises notably leukemias, seminomas, melanomas, teratomas, lymphomas, neuroblastomas, gliomas and sarcomas. The term cancer notably include rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer, soft tissue tumors and the metastases thereof. The term cancer according to the present disclosure also comprises cancer metastases and relapse of cancer.

The term "Growth of a tumor" or "tumor growth" according to the present disclosure relates to the tendency of a tumor to increase its size and/or to the tendency of tumor cells to proliferate.

For purposes of the present disclosure, the terms "cancer" and "cancer disease" are used interchangeably with the terms "tumor" and "tumor disease".

By "treatment" or "treat", it is meant both prophylactic or preventive treatment as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "treat" is meant to administer a compound or composition as described herein to a subject in order to prevent or eliminate a disease, including reducing the size of a tumor or the number of tumors in a subject; arrest or slow a disease in a subject; inhibit or slow the development of a new disease in a subject; decrease the frequency or severity of symptoms and/or recurrences in a subject who currently has or who previously has had a disease; and/or prolong, i.e. increase the lifespan of the subject. In particular, the term "treatment of a disease" includes curing, shortening the duration, ameliorating, preventing, slowing down or inhibiting progression or worsening, or preventing or delaying the onset of a disease or the symptoms thereof.

By "therapeutic regimen" is meant the pattern of treatment of an illness, e.g., the pattern of dosing used during therapy. A therapeutic regimen may include an induction regimen and a maintenance regimen.

The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both.

The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

The therapeutically active agents, products or compositions described herein may be administered via any conventional route, including by injection or infusion.

By a "therapeutically effective amount", it is meant a sufficient amount of products and compositions according to the invention, to treat the disease (e.g. cancer) at a reasonable benefit/risk ratio applicable to any medical treatment. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

It will be understood that the total daily usage of the product of the present invention will be decided by the attending physician within the scope of sound medical judgment. The specific therapeutically effective dose level for any particular patient will depend on the condition to be treated, the severity of the disease, the individual parameters of the patient, including age, body size and weight, general health, sex, diet of the patient, physiological condition, the time of administration, route of administration, and rate of excretion of the specific compound employed; the duration of treatment, the type of an accompanying therapy (if present), drugs used in combination or coincidental with the product; and like factors well known in the medical arts. For example, it is well known within the skill of the art to start doses of the compound at levels lower than those required to achieve the desired therapeutic effect and to gradually increase the dosage until the desired effect is achieved. Accordingly, the doses administered of the agents described herein may depend on several of such parameters. In the case that a reaction in a patient is insufficient with an initial dose, higher doses (or effectively higher doses achieved by a different, more localized route of administration) may be used.

By "pharmaceutical" or "pharmaceutically acceptable", it is meant that molecular entities and compositions do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings.

The pharmaceutical compositions as herein described are preferably sterile and contain an effective amount of the therapeutically active substance to generate the desired reaction or the desired effect.

The pharmaceutical compositions as herein described are generally administered in pharmaceutically compatible amounts and in pharmaceutically compatible preparation. The term "pharmaceutically compatible" refers to a nontoxic material which does not interact with the action of the active component of the pharmaceutical composition. Preparations of this kind may usually contain salts, buffer substances, preservatives, carriers, supplementing immunity-enhancing substances such as adjuvants, e.g. CpG oligonucleotides, cytokines, chemokines, saponin, GM-CSF and/or RNA and, where appropriate, other therapeutically active compounds. When used in medicine, the salts should be pharmaceutically compatible.

Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The product can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The term "immune cell" has its general meaning in the art and refers to cells derived from the blood, bone marrow, lymph, or lymphoid organs (notably the thymus) and are cells of the immune system (i.e., immune cells), such as cells of the innate or adaptive immunity, e.g., myeloid or lymphoid cells, including monocytes, macrophages, lymphocytes, T cells (CD4+ or CD8+ T cell), NK cells, lymphoid progenitors and/or T cell progenitors.

Typically also, the engineered immune cell especially the T cell or myeloid cell is isolated from a subject. Preferably, said subject is suffering from a cancer or is at risk of suffering from a cancer.

The cells typically are primary cells, such as those isolated directly from a subject and/or isolated from a subject and frozen.

With reference to the subject to be treated, the cells of the invention may be allogeneic and/or autologous.

The cells and compositions containing the cells according to the invention are isolated from a sample, notably a biological sample, e.g., obtained from or derived from a subject. Typically, the subject needs a cell therapy (adoptive cell therapy) and/or will receive the cell therapy. Samples include, in the context of cell therapy (typically adoptive cell therapy) samples from autologous and allogeneic sources. In some embodiments, the cells are derived from cell lines. The cells can also be obtained from a xenogeneic source, such as a mouse, a rat, a non-human primate, or a pig. Preferably, the cells are human cells.

### Single domain antibodies and variants thereof

As used herein, the term "HER2" has its general meaning in the art and includes human HER2 (also named "Receptor tyrosine-protein kinase erbB-2"), in particular the native-sequence polypeptide, isoforms, chimeric polypeptides, all homologs, fragments, and precursors of human HER2. The amino acid sequence for native HER2 includes the UniProt reference P04626 (ERBB2_HUMAN).

More specifically the term "HER2" includes the human HER2 of the following SEQ ID NO:1.
>sp|P04626|ERBB2_HUMAN Receptor tyrosine-protein kinase erbB-2 OS=Homo sapiens OX=9606 GN=ERBB2 PE=1 SV=1 (SEQ ID NO:1)

The term "antibody", broadly refers to any immunoglobulin (Ig) molecule, or antigen binding portion thereof, comprised of four polypeptide chains, two heavy (H) chains and two light (L) chains, or any functional fragment, mutant, variant, or derivation thereof, which retains the essential epitope binding features of an Ig molecule. Such mutant, variant, or derivative antibody formats are known in the art. In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Immunoglobulin molecules can be of any type (e.g., IgG, IgE, IgM, IgD, IgA and IgY), class (e.g., IgG1, lgG2, IgG 3, IgG4, IgAI and IgA2) or subclass.

An antibody fragment is a portion of an antibody, for example as F(ab')2, Fab, Fv, sFv and the like. Functional fragments of a full length antibody retain the target specificity of a full length antibody. Recombinant functional antibody fragments, such as Fab (Fragment, antibody), scFv (single chain variable chain fragments) and single domain antibodies (dAbs) have therefore been used to develop therapeutics as an alternative to therapeutics based on mAbs. scFv fragments (~25kDa) consist of the two variable domains, VH and VL. Naturally, VH and VL domains are non-covalently associated via hydrophobic interaction and tend to dissociate. However, stable fragments can be engineered by linking the domains with a hydrophilic flexible linker to create a single chain Fv (scFv). The smallest antigen binding fragment is the single variable fragment, namely the VH or VL domain. Binding to a light chain/heavy chain partner respectively is not required for target binding. Such fragments are used in single domain antibodies. A single domain antibody (-12 to 15 kDa) therefore has either the VH or VL domain.

As used herein the term "single-domain antibody" (sdAb) or nanobody^{®} (tradename of Ablynx). has its general meaning in the art and refers to an antibody fragment with a molecular weight of only 12-15 kDa consisting of a single monomeric variable antibody domain derived from a heavy chain. Such single domain antibody (named VHH) can be found in Camelid mammals and are naturally devoid of light chains. For a general description of (single) domain antibodies, reference is also made to the prior art cited above, as well as to EP 0 368 684, Ward et al. (Nature 1989 Oct 12; 341 (6242): 544-6), Holt et al, Trends Biotechnol, 2003, 21(11):484-490; and WO 06/030220, WO 06/003388. The amino acid sequence and structure of a single-domain antibody can be considered to be comprised of four framework regions or "FRWs" which are referred to in the art and herein as "Framework region 1" or "FRW1"; as "Framework region 2" or "FRW2"; as "Framework region 3 " or "FRW3"; and as "Framework region 4" or "FRW4" respectively; which framework regions are interrupted by three complementary determining regions or "CDRs", which are referred to in the art as "Complementary Determining Region 1" or "CDR1"; as "Complementarity Determining Region 2" or "CDR2" and as "Complementarity Determining Region 3" or "CDR3", respectively. Accordingly, the single-domain antibody can be defined as an amino acid sequence with the general structure : FRW1 - CDR1 - FRW2 - CDR2 - FRW3 - CDR3 - FRW4 in which FRW1 to FRW4 refer to framework regions 1 to 4 respectively, and in which CDR1 to CDR3 refer to the complementarity determining regions 1 to 3. In the context of the present disclosure, the amino acid residues of the single-domain antibody are numbered according to the general numbering for VH domains given by the International ImMunoGeneTics information system aminoacid numbering (http://imgt.cmes .fr/).

An "isolated sdAb", as used herein, refers to a single domain antibody (sdAb) that is substantially free of other antibodies, notably other sdAb having different antigenic specificities (e.g., an isolated antibody that specifically binds to HER2 is substantially free of antibodies that specifically bind to other antigens than HER2). Moreover, an isolated antibody may be substantially free of other cellular material and/or chemicals.

As used herein, the term "synthetic" means that such antibody has not been obtained from fragments of naturally occurring antibodies but produced from recombinant nucleic acids comprising artificial coding sequences.

The term "CDR" refers to the complementarity-determining region within antibody variable sequences. There are three CDRs in each of the variable regions of the heavy chain and the light chain, which are designated CDR1, CDR2 and CDR3, for each of the variable regions. The term "CDR set" refers to a group of three CDRs that occur in a single variable region capable of binding the antigen. The exact boundaries of these CDRs have been defined differently according to different systems. The system described by Kabat is used herein. The terms "Kabat numbering", "Kabat definitions" and "Kabat labeling" are used interchangeably herein. These terms, which are recognized in the art, refer to a system of numbering amino acid residues which are more variable (i.e., hypervariable) than other amino acid residues in the heavy and light chain variable regions of an antibody, or an antigen binding portion thereof (Kabat et al., (1971) Ann. NY Acad. Sci. 190:382-391 and Kabat, et al., (1991) Sequences of Proteins of Immunological Interest, Fifth Edition, U.S. Department of Health and Human Services, NIH Publication No. 91-3242).

sdAb affinity refers to the strength with which the sdAb binds to the epitope presented on an antigen, such as a HER2 in the present disclosure, through its antigen-binding site (paratope). Affinity may be assessed based on assessment of the K_{D} value.

The term "K_{D}", as used herein, is intended to refer to the equilibrium dissociation constant, which is obtained from the ratio of k_{off} to kₒₙ (i.e. k_{off}/kₒₙ) and is expressed as a molar concentration (M). The K_{D} value relates to the concentration of antibody (the amount of antibody needed for a particular experiment) and so the lower the K_{D} value (lower concentration) and thus the higher the affinity of the antibody. K_{D} values for antibodies can be determined using methods well established in the art. Methods for determining the K_{D} values of mAbs can be found in Harlow et al., Antibodies: A Laboratory Manual, Cold Spring Harbor Laboratory Press, Cold Spring Harbor, N.Y., 1988; Coligan et al., eds., Current Protocols in Immunology, Greene Publishing Assoc. and Wiley Interscience, N.Y., 1992, 1993, and Muller, Meth. Enzymol. 92:589-601, 1983, which references are entirely incorporated herein by reference. A method for determining the K_{D} of an antibody is by using surface plasmon resonance, or by using a biosensor system such as a Biacore^{®} (see also for detailed information regarding affinity assessment Rich RL et al., Anal Biochem, 2001, but also for more details about the specific implementation of affinity measurement for sdAb Moutel S et al., eLife 2016;5:e16228). Affinity measurements are generally performed at 25°C. The terms "k_{assoc}" or "ka", or "kₒₙ" as used herein, is intended to refer to the association rate of a particular antibody-antigen interaction, whereas the terms "k_{dis}" or "k_{d},", or k_{off} as used herein, is intended to refer to the dissociation rate of a particular antibody-antigen interaction. Briefly, as sdAb are smaller proteins that their respective antigens, they can be capture on a sensorship from a Biocore biosensor instrument, while the recombinant antigens (i.e., typically rHER2) can be used as analytes. Analytes can be injected sequentially with increased concentration ranging for example between 3.125 nM to 50 nM in a single cycle without regeneration of the sensorship between injections. Binding parameters can be obtained by fitting the overlaid sensorgrams with the 1:1. Langmuir binding model of the BIAevalutation software.

Affinity measurement can also be performed with the bio-layer interferometry (BLI) based Octet biosensor (see also the results for more details). The principle of BLI technology is based on the optical interference pattern of white light reflected from two surfaces - a layer of immobilized protein and an internal reference layer. The binding between a ligand immobilized on the biosensor tip surface and an analyte in solution produces an increase in optical thickness at the biosensor tip, which results in a shift in the interference pattern measured in nanometers. The wavelength shift (Δλ) is a direct measure of the change in optical thickness of the biological layer, when this shift is measured over a period of time and its magnitude plotted as a function of time, a classic association/dissociation curve is obtained. This interaction is measured in real-time, providing the ability to monitor binding specificity, association rate and dissociation rate, and concentration with outstanding precision and accuracy.

In some embodiments, the apparent affinity may be assessed in binding assays using an ELISA assay (with typically hHER2-Fc coated wells) or flow cytometry (typically using cells expressing recombinant HER2, notably hHER2)

Typically, a single domain antibody as per the present disclosure binds to HER2, notably human HER2 as herein defined with a K_{D} binding affinity of about 10⁻⁶ M or less, 10⁻⁷ M or less, 10⁻⁸ M or less, 10⁻⁹ M or less, 10⁻¹⁰ M or less, or 10⁻¹¹ M or less. In some embodiments, the K_{D} binding affinity is in the nano/pico-molar range, notably comprised between 10⁻⁷ and 10⁻¹² M, notably between 10⁻⁸ and 10⁻¹², in particular between 10⁻⁸ and 10⁻¹⁰.

The inventors have isolated 4 reference single-domain antibodies (sdAb) with the required properties, notably the required affinity and characterized by following sequences:
>VH anti-Her2 (SEQ ID NO:2) 
MAEVQLVESGGGLVQPGGSLRLSCAASGAFYKAYNMGWVRQAPGKGLEWV
SAISATQGKWVYYADSVKGRFTISRDNSKNTVYLQMNSLRAEDTAVYYCAKLPISKK
ALYRGQGTLVTVSS
>FRW1 (SEQ ID NO:3)
   MAEVQLVESGGGLVQPGGSLRLSCAASG
>FRW2 (SEQ ID NO:4)
   MGWVRQAPGKGLEWVSAIS
>FRW3 (SEQ ID NO:5)
   YYADSVKGRFTISRDNSKNTVYLQMNSLRAEDTAVYYCA
>FRW4 (SEQ ID NO:6)
   YRGQGTLVTVSS
>CDR1 (SEQ ID NO:7)
   AFYKAYN
>CDR2 (SEQ ID NO:8)
   ATQGKWV
>CDR3 (SEQ ID NO:9)
   KLPISKKAL

Therefore, the present disclosure encompasses single domain antibody having at least the 3 CDRs as defined in SEQ ID NO:2-9.

sdAb as detailed in SEQ ID NO:2 comprise framework regions including humanized amino acid residues and is therefore referred as humanized sdAb (hsdAb) or is also named humanized synthetic sdAb (hssdAb).

In some embodiments, sdAb according to the present disclosure include sdAbs having at least 60, 70, 80, 90, 95, 96, 97, 98, 99 or 100 percent identity with the amino acid sequences as set forth in any one of SEQ ID NO:2-9.

sdAbs as per the present disclosure notably include humanized anti-HER2 sdAbs having framework region sequences that have at least 60, 70, 80, 90, 95, 96, 97, 98, 99 or 100 percent identity with one or more of the sequences SEQ ID NO:2-9.

In some embodiments of the present invention, the 3 CDR regions of humanized anti-HER2 sdAbs as herein disclosed can be 100% identical to the 3 CDR regions of the reference humanized sdAb (hsdAb) of the invention. Alternatively, in some embodiments, hsdAbs according to the present disclosure may have an amino acid sequence that have been mutated by amino acid deletion, insertion or substitution, yet that have at least 60, 70, 80, 90, 95, 96, 97, 98, 99 or 100 percent identity in the CDR regions compared with the CDR regions of the sdAb of the invention. Typically, as per the present disclosure, antibodies may have between 1, 2, 3 or 4 amino acid variations (including deletion, insertion or substitution) in one or more CDRs, as compared to the respective CDR sequences of the sdAb of the invention.

In some embodiments, the single domain antibody of the present disclosure is a mutant variant of the reference single domain antibody of the invention, having the 3 CDR regions 100% identical to the corresponding 3 CDR regions of said reference sdAb, and wherein no more than 1, 2, 3, 4 or 5 amino acids have been mutated by amino acid deletion, insertion or substitution in one or more of the FRW1, FRW2, FRW3 and/or FRW4 regions, when compared with the corresponding framework regions of the corresponding reference sdAb.

In some embodiments, a sdAb of the present disclosure is sdAb having one or more amino acid substitutions, deletions, insertions or other modifications compared to the sdAb of the invention, and which retains a biological function of the single domain antibody. Modifications may include one or more substitution, deletion or insertion of one or more codons encoding the single domain antibody or polypeptide that results in a change in the amino acid sequence as compared with the sequence of the reference single domain antibody or polypeptide. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

In some embodiments, the modification is a conservative sequence modification. As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into single domain antibody as herein described by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of a single domain antibody of the present disclosure can be replaced with other amino acid residues from the same side chain family and the altered antibody can be tested for retained function (i.e., the functions set forth in (c) through (I) above) using the functional assays described herein.

In some embodiments, the single domain antibody comprises one or more amino acid substitutions, for example 1 to 20, such as 1 , 2, 3, 4, 5, 6, 7, 8, 9 or 10 amino acid substitutions. The one or more amino acid substitution can be in one or more of the framework areas. Alternatively, or in addition, the one or more amino acid substitution can be in one or more of the CDRs. In some embodiments, the amino acid substitutions are in the framework and CDR sequences.

In some embodiments, the humanized single domain antibody is a variant of the single domain antibody of the invention, that comprises one or more sequence modification and has improvements in one or more of a property such as binding affinity, specificity, thermostability, expression level, effector function, glycosylation, reduced immunogenicity, or solubility as compared to the unmodified single domain antibody.

In preferred embodiments, a sdAb according to the present disclosure has strict specificity for HER2. By having strict specificity for HER2, it is herein intended that the sdA according to the present disclosure show no detectable binding to other HER molecules such as HER1, HER3, or HER4. Binding assay can be performed as illustrated in the results by detecting sdAb binding to cell expressing of not the various HER molecules at their surface. Typically, the assay can be performed by flow cytometry using a labelled (for example a FITC-labelled anti-iXHis-tag antibody) to detect surface-bound single domain antibodies.

Typically, a single domain antibody as per the present disclosure has an affinity (K_{D}) for HER2 which is at least of 10⁻⁷, notably at least of 10⁻⁸, at least of 10⁻⁹ M, while having no affiniy, or a very low affinity for other HER molecules (such as HER1, HER3, and/or HER4) of less than 10⁻³ M. In some embodiment a sdAb specific for HER2 has no affinity for HER1, HER3, and HER4.

In preferred embodiments, sdAb as per the present disclosure inhibit HER2 signalling. Analysis of this properties can be performed as illustrated in the results enclosed herein.

A skilled person will know that there are different ways to identify, obtain and optimise the antigen binding molecules as described herein, including *in vitro* and *in vivo* expression libraries. Optimisation techniques known in the art, such as display (e.g., ribosome and/or phage display) and / or mutagenesis (e.g., error-prone mutagenesis) can be used. The present disclosure therefore also comprises sequence optimised variants of the single domain antibodies described herein.

In some embodiments, sdAb according to the present disclosure can be chemically modified, for example to increase their molecular weight to reduce renal clearance or protect for example from proteases. PEGylation (covalent attachment of a polyethylene glycol (PEG) group) for example has been widely used to increase the half-life. Other strategies to limit renal clearance involve attachment of negative charges to the sdAb, such as addition of sialic acid polymers (polysialylation) or hydroxyethal starch (HESylation) and by fusion with the highly syaliated beta carboxy terminal peptide (CTP) amino acid-residue of the human chorionic gonadotrophin (hCG) hormone.

In some embodiments of the present disclosure, an isolated humanized single domain antibody as herein described can be linked directly or not, covalently or not to a compound of interest. The substance or compound of interest as defined above can be directly and covalently or non-covalently linked to a single domain antibody as herein defined either to one of the terminal ends (N or C terminus), or to the side chain of one of the amino acids of said single domain antibody. The substance of interest can also be indirectly and covalently or non-covalently linked to said single domain antibody by means of a spacer (or linker) either to one of the terminal ends of said single domain antibody, or to a side chain of one of the amino acids of said single domain antibody. Conventional linking methods of a substance of interest to a peptide, in particular an antibody, are known in the art (e.g., See Ternynck and Avrameas 1987 "Techniques immunoenzymatiques" Ed. INSERM, Paris; Hermanson, 2010, Bioconjugate Techniques, Academic Press).

In some embodiments, single domain antibody as herein described can be notably in the form of "antibody drug conjugate" of the formula sdAb-(L-(D)m)n or a pharmaceutically acceptable salt thereof; wherein sdAb is a single domain antibody as previously disclosed; L is a linker; D is a compound of interest; m is an integer from 1 to 8; and n is an integer from 1 to 10, typically 3 or 4.

The term "antibody drug conjugate" as used herein refers to the linkage of a single domain antibody with another agent, such as a chemotherapeutic agent, a toxin, an immunotherapeutic agent, an imaging probe, and the like. The linkage can be covalent bonds, or non-covalent interactions such as through electrostatic forces. Various linkers, known in the art, can be employed in order to form the immunoconjugate. The linker (L) can be for example selected from the group consisting of a cleavable linker, a non-cleavable linker, a hydrophilic linker, a procharged linker and a dicarboxylic acid-based linker.

In some embodiments, the single domain antibody of the present disclosure is conjugated, or covalently linked to the compound of interest. As used herein, the term "conjugation" has its general meaning in the art and means a chemical conjugation, or chemical crosslinking. Many chemical cross-linking methods are also known in the art. Cross-linking reagents may be homobifunctional (i.e., having two functional groups that undergo the same reaction) or heterobifunctional (i.e., having two different functional groups). Numerous cross-linking reagents are commercially available. Detailed instructions for their use are readily available from the commercial suppliers. A general reference on polypeptide cross-linking and conjugate preparation is: WONG, Chemistry of protein conjugation and cross-linking, CRC Press (1991), see also Arnon et al., "Monoclonal Antibodies For Immunotargeting Of Drugs In Cancer Therapy," in Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Deiker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62: 119-58. See also, e.g., PCT publication WO 89/12624.). Typically, the nucleic acid molecule is covalently attached to lysines or cysteines on the antibody, through N-hydroxysuccinimide ester or maleimide functionality respectively. Methods of conjugation using engineered cysteines or incorporation of unnatural amino acids have been reported to improve the homogeneity of the conjugate (Axup, J.Y., Bajjuri, K.M., Ritland, M., Hutchins, B.M., Kim, C.H., Kazane, S.A., Haider, R., Forsyth, J.S., Santidrian, A.F., Stafin, K., et al. (2012). Synthesis of site-specific antibody-drug conjugates using unnatural amino acids. Proc. Natl. Acad. Sci. USA 109, 16101-16106.; Junutula, J.R., Flagella, K.M., Graham, R.A., Parsons, K.L., Ha, E., Raab, H., Bhakta, S., Nguyen, T., Dugger, D.L., Li, G., et al. (2010) Engineered thio-trastuzumab-DMl conjugate with an improved therapeutic index to target human epidermal growth factor receptor 2-positive breast cancer. Clin. Cancer Res. 16, 4769-4778.). Junutula et al. (2008) developed cysteine-based site-specific conjugation called "THIOMABs" (TDCs) that are claimed to display an improved therapeutic index as compared to conventional conjugation methods. In particular the one skilled in the art can also envisage a polypeptide engineered with an acyl donor glutamine-containing tag (e.g., Gin-containing peptide tags or Q-tags) or an endogenous glutamine that are made reactive by polypeptide engineering (e.g., via amino acid deletion, insertion, substitution, or mutation on the polypeptide). Then a transglutaminase, can covalently crosslink with an amine donor agent (e.g., a small molecule comprising or attached to a reactive amine) to form a stable and homogenous population of an engineered Fc-containing polypeptide conjugate with the amine donor agent being site-specifically conjugated to the Fc-containing polypeptide through the acyl donor glutamine- containing tag or the accessible/exposed/reactive endogenous glutamine (WO 2012059882). The term "transglutaminase", used interchangeably with "TGase" or "TG", refers to an enzyme capable of cross-linking proteins through an acyl-transfer reaction between the γ-carboxamide group of peptide-bound glutamine and the ε-amino group of a lysine or a structurally related primary amine such as amino pentyl group, e.g. a peptide-bound lysine, resulting in a ε-(γ-glutamyl) lysine isopeptide bond. TGases include, inter alia, bacterial transglutaminase (BTG) such as the enzyme having EC reference EC 2.3.2.13 (protein-glutamine-y-glutamyltransferase). In some embodiments, the single domain antibody of the present disclosure is conjugated to the heterologous moiety by a linker molecule. As used herein, the term "linker molecule" refers to any molecule attached to the single domain antibody the present disclosure. The attachment is typically covalent. In some embodiments, the linker molecule is flexible and does not interfere with the binding of the single domain antibody the present disclosure.

A compound or substance of interest as herein intended can be non-limitatively selected from a nucleic acid, a polypeptide or a protein, a virus, a toxin and a chemical entity.

In some embodiments, compounds of interest include antigen binding domain agents such as antibodies, variants and fragments thereof, notably the same or another single domain antibody, aptamers, or enzymes.

The compound or substance of interest, as above described, can be a therapeutic or a diagnostic compound. Therapeutic compounds notably include therapeutic compounds having anti-cancer and/or cytotoxic activity (i.e. cytotoxic compounds), and diagnostic compounds typically include imaging probes.

Chemotherapeutic drugs often lack specificity and can also affect healthy tissues. To overcome this problem, nano vehicles can be used to deliver drugs actively to the tumor site. The use of nanotechnology in the field of chemotherapy has the potential to improve biodistribution by increasing drug concentrations at the tumor site and reduce toxicity to normal cells (see Ferrari, M. Cancer nanotechnology: Opportunities and challenges. Nat. Rev. Cancer 5, 161-171 (2005); and Kumari, P., Ghosh, B. & Biswas, S. Nanocarriers for cancer-targeted drug delivery. J. Drug Target. 24, 179-191 (2016) for detailed reviews). Thus, in some embodiments, said substance of interest is a drug nanocarrier, that can be organic such as liposomes or a polymeric entities or inorganic comprising, or encapsulating, a diagnostic or therapeutic compound (Villaraza et al. 2010 Chem Rev., 110, 2921-2959). Hence, nanobodies are very convenient tools for delivering toxic cargos to cancer cells and are well-suited for chemical conjugation onto different nanoparticle or nanocarrier formats. Organic carriers (or cargos) may include lipoparticles such as liposomes or micelles, albumin-based nanoparticles and polymeric nanoparticules including polymer-based polymersomes. Inorganic carriers may include quantum dots, carbon nanotubes, layered double hydroxides, mesoporous silica and magnetic nanoparticles (see notably Senapati, S., Mahanta, A.K., Kumar, S. et al. Controlled drug delivery vehicles for cancer treatment and their performance. Sig Transduct Target Ther 3, 7 (2018)).

Polymeric nanoparticles are solid, biocompatible, colloidal and often biodegradable systems with nanoscale dimensions. Polymeric nanoparticles can be made from synthetic polymers, e.g., poly(lactic acid) (PLA), poly(ε-caprolactone) (PCL), poly(lactic-co-glycolic acid), N-(2-hydroxypropyl)-methacrylamide copolymer (HPMA) and poly(styrene-maleic anhydride) copolymer, or from natural polymers, such as gelatin, dextran, guar gum, chitosan, and collagen. Drugs can easily be encapsulated either through dispersion in the polymer matrix or conjugation/attachment to polymer molecules for their controlled delivery through surface or bulk erosion, diffusion through the polymer matrix, swelling followed by diffusion, or as a response to local stimuli.

Albumin is a protein that can be obtained from a variety of sources, including egg white (ovalbumin), bovine serum (bovine serum albumin, BSA), and human serum (human serum albumin, HSA), and is available in soybeans, milk, and grains. Albumin-based nanocarriers have several advantages, such as easy preparation, a high binding capacity for various drugs, nontoxic, non-immunogenic, biocompatible, and biodegradable properties, and along half-life in circulating plasma. The presence of functional groups (amino and carboxylic groups) on albumin nanoparticles surfaces makes it easy to bind targeting ligands and other surface modifications.

Micelles are spherical or globular colloidal nanoscale systems formed by self-assembly of amphiphilic block copolymers in an aqueous solution, resulting in a hydrophobic core and a hydrophilic shell. They belong to a group of amphiphilic colloids that can be formed spontaneously under certain concentrations (critical micelle concentration; CMC) and temperatures. The hydrophobic core serves as a reservoir for hydrophobic drugs, whereas the hydrophilic shell stabilizes the hydrophobic core and renders both polymer and hydrophobic drugs water soluble, making the particle an appropriate candidate for i.v. administration. The drugs are incorporated into a polymeric micelle through physical, chemical, or electrostatic interactions.

Liposomes are small, spherical, self-closed structures with at least one concentric lipid bilayer and an encapsulated aqueous phase in their core. They have been widely used as drug delivery vehicles since their discovery in 1965, due to their biocompatible and biodegradable nature and their unique ability to encapsulate hydrophilic agents (hydrophilic drugs, DNA, RNA, etc.) in their inner aqueous core and hydrophobic drugs within the lamellae, which makes them versatile therapeutic carriers. Furthermore, amphiphilic drugs can also be loaded into the liposome inner aqueous core using remote loading methods, such as the ammonium sulfate method for doxorubicin or the pH gradient method for vincristine (see Bolotin, E. M. et al. Ammonium sulfate gradients for efficient and stable remote loading of amphipathic weak bases into liposomes and ligandoliposomes. J. Liposome. Res. 4, 455-479 (1994), and Boman, N. L. et al., Liposomal vincristine which exhibits increased drug retention and increased circulation longevity cures mice bearing P388 tumors. Cancer Res. 54, 2830-2833 (1994)). Ligand-targeted liposomes have been found to promote the internalization of liposome-drug conjugates into specific target cells both in vitro and in vivo. Some liposomal formulations of chemotherapeutics have been translated into the clinic and demonstrated safety and improved pharmacokinetic properties of the drug. Prominent examples are liposomal doxorubicin (Doxil^{®}), daunorubicin (DaunoXome^{®}) and VCR (Marqibo^{®}) which have contributed to reduced side-effects compared to the free drug (see O'Brien, M. E. R. et al., Ann. Oncol. 15, 440-449 (2004); Gill, P. S. et al., J. Clin. Oncol. 14, 2353-2364 (1996); and Shah, N. N. et al., Pediatr. Blood Cancer 63, 997-1005 (2016)).

For a recent review on liposome technologies for delivery of therapeutic compounds, see Bulbake, Upendra et al. "Liposomal Formulations in Clinical Use: An Updated Review." Pharmaceutics vol. 9,2 12. 27 Mar. 2017. A typical example of targeted liposomes (i.e. bound to sdAbs) is notably described in Roveri, M. et al., Nanomedicine nnm-2017-0430 (2017). Exemplified liposome notably comprises a combination of egg sphingomyelin, cholesterol, PEG-ceramide (typically N-palmitoyl-sphingosine-1-[succinyl[methoxyPEG-2000]]), DSPE-PEG-maleimide (typically 1,2-distearoyl-sn-glycero-3-phosphoethanolamine-N-[maleimide (polyethylene glycol)-2000]) (both Avanti Polar Lipids) and DiR (1,1'-dioctadecyl-3,3,3',3'-tetramethylindotricarbocyanine Iodide) (Thermo Fisher Scientific). A favoured ratio of said lipids is typically of 49.8:45:4:1:0.2 mol%, respectively. A concentration around 70 mM of total lipids is achieved in the lipid film.

To produce actively targeted liposomes, DSPE-PEG lipids were introduced with reactive maleimide groups at the distal end. Single domain antibodies harboring a free cysteine at the C-terminus were then coupled to the liposomal surface. Typical therapeutic agents that can be encapsulated into liposomes include doxorubicin (Doxil^{®}, Myocet^{®}), daunorubicin (DaunoXome^{®}), VCR (Marqibo^{®}), Vinorelbine (TCL178).

The compounds as listed below can be directly conjugated to a single domain antibody as herein disclosed or encapsulated into a carrier as described above.

The term "toxin," "cytotoxin" or "cytotoxic compound" as used herein, refers to any agent that is detrimental to the growth and proliferation of cells and may act to reduce, inhibit, or destroy a cell or malignancy.

The term "anti-cancer compound" as used herein refers to any agent that can be used to treat a cell proliferative disorder such as cancer, including but not limited to, cytotoxic agents, chemotherapeutic agents, radioisotopes, targeted anti-cancer agents, immunotherapeutic agents (such as immunosuppressants or immune stimulators), and lytic peptides.

A therapeutic compound having anti-cancer or cytotoxic activity can be for example selected from a group consisting of a V-ATPase inhibitor, a pro-apoptotic agent, a Bcl2 inhibitor, an MCL1 inhibitor, a HSP90 inhibitor, an IAP inhibitor, an mTor inhibitor, a microtubule stabilizer, a microtubule destabilizer, an auristatin, a dolastatin, a maytansinoid, a MetAP (methionine aminopeptidase), an inhibitor of nuclear export of proteins CRM1 , a DPPIV inhibitor, proteasome inhibitors, inhibitors of phosphoryl transfer reactions in mitochondria, a protein synthesis inhibitor, a kinase inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder and a DHFR inhibitor.

Cytotoxic compound may, for example, be selected from the group consisting of taxol; cytochalasin B; gramicidin D; ethidium bromide; emetine; mitomycin; etoposide; tenoposide; vincristine; vinblastine; colchicin; doxorubicin; daunorubicin; dihydroxyanthracindione; a tubulin-inhibitor such as maytansine or an analog or derivative thereof; an antimitotic agent such as mono methyl auristatin E or F or an analog or derivative thereof; dolastatin 10 or 15 or an analogue thereof; irinotecan or an analogue thereof; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite such as methotrexate, 6 mercaptopurine, 6 thioguanine, cytarabine, fludarabin, 5 fluorouracil, decarbazine, hydroxyurea, asparaginase, gemcitabine, or cladribine; an alkylating agent such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C; a platinum derivative such as cisplatin or carboplatin; duocarmycin A, duocarmycin SA, rachelmycin (CC-1065), or an analog or derivative thereof; an antibiotic such as dactinomycin, bleomycin, daunorubicin, doxorubicin, idarubicin, mithramycin, mitomycin, mitoxantrone, plicamycin, anthramycin (AMC)); pyrrolo[2,1-c][1,4]-benzodiazepines (PDB); diphtheria toxin and related molecules such as diphtheria A chain and active fragments thereof and hybrid molecules, ricin toxin such as ricin A or a deglycosylated ricin A chain toxin, cholera toxin, a Shiga- like toxin such as SLT I, SLT II, SLT IIV, LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleuritesfordii proteins, dianthin proteins, Phytolaccaamericana proteins such as PAPI, PAPII, and PAP-S, momordicacharantia inhibitor, curcin, crotin, sapaonariaofficinalis inhibitor, gelonin, mitogellin, restrictocin, phenomycin, and enomycin toxins; ribonuclease (R ase); DNase I, Staphylococcal enterotoxin A; pokeweed antiviral protein; diphtherin toxin; and Pseudomonas endotoxin.

In some embodiments, the therapeutic compound conjugated to a sdAb as herein disclosed or encapsulated into a nanocarrier functionalized with an sdAb as herein disclosed include an auristatin or a peptide analog, derivative or prodrug thereof. Auristatins have been shown to interfere with microtubule dynamics, GTP hydrolysis and nuclear and cellular division (Woyke et al (2001) Antimicrob. Agents and Chemother. 45(12): 3580-3584) and have anti-cancer (US5663149) and antifungal activity (Pettit et al, (1998) Antimicrob. Agents and Chemother. 42: 2961-2965). For example, auristatin E can be reacted with para-acetyl benzoic acid or benzoylvaleric acid to produce AEB and AEVB, respectively. Other typical auristatin derivatives include AFP, MMAF (monomethylauristatin F), and MMAE (monomethylauristatin E). Suitable auristatins and auristatinanalogs, derivatives and prodrugs, as well as suitable linkers for conjugation of auristatins to Abs, are described in, e.g., U.S. Patent Nos. 5,635,483, 5,780,588 and 6,214,345 and in International patent application publications WO02088172, WO2004010957, WO2005081711, WO2005084390, WO2006132670, WO03026577, WO200700860, WO207011968 and WO205082023.

In some embodiments, the therapeutic compound conjugated to a sdAb as herein disclosed or encapsulated into a nanocarrier functionalized with an sdAb as herein disclosed include Mertansine (also called emtansine or DM1) or a peptide analog, derivative or prodrug thereof. Mertansine is a tubulin inhibitor, meaning that it inhibits the assembly of microtubules by binding to tubulin.

In some embodiments, the therapeutic compound conjugated to a sdAb as herein disclosed or encapsulated into a nanocarrier functionalized with an sdAb as herein disclosed include pyrrolo[2,l-c][l,4]-benzodiazepine (PDB) or an analog, derivative or prodrug thereof. Suitable PDBs and PDB derivatives, and related technologies are described in, e.g., Hartley J. A. et al, Cancer Res 2010; 70(17) : 6849-6858; Antonow D. et al, Cancer J 2008; 14(3) : 154-169; Howard P.W. et al, Bioorg Med ChemLett 2009; 19: 6463-6466 and Sagnou et al, Bioorg Med ChemLett 2000; 10(18) : 2083-2086.

In some embodiments, the therapeutic compound conjugated to a sdAb as herein disclosed or encapsulated into a nanocarrier functionalized with an sdAb as herein disclosed include the group consisting of an anthracycline, maytansine, calicheamicin, duocarmycin, rachelmycin (CC-1065), dolastatin 10, dolastatin 15, irinotecan, monomethylauristatin E, monomethylauristatin F, a PDB, or an analog, derivative, or prodrug of any thereof.

In some embodiments, the therapeutic compound conjugated to a sdAb as herein disclosed or encapsulated into a nanocarrier functionalized with an sdAb as herein disclosed include an anthracycline or an analog, derivative or prodrug thereof. In some embodiments, the single domain antibody is conjugated to maytansine or an analog, derivative or prodrug thereof. In some embodiments, the single domain antibody is conjugated to calicheamicin or an analog, derivative or prodrug thereof. In some embodiments, the single domain antibody is conjugated to duocarmycin or an analog, derivative or prodrug thereof. In some embodiments, the single domain antibody is conjugated to rachelmycin (CC-1065) or an analog, derivative or prodrug thereof. In some embodiments, the antibody is conjugated to dolastatin 10 or an analog, derivative or prodrug thereof. In some embodiments, the antibody is conjugated to dolastatin 15 or an analog, derivative or prodrug thereof. In some embodiments, the antibody is conjugated to monomethylauristatin E or an analog, derivative or prodrug thereof. In some embodiments, the single domain antibody is conjugated to monomethylauristatin F or an analog, derivative or prodrug thereof. In some embodiments, the antibody is conjugated to pyrrolo[2,l-c][l,4]-benzodiazepine or an analog, derivative or prodrug thereof. In some embodiments, the single domain antibody is conjugated to irinotecan or an analog, derivative or prodrug thereof.

In some embodiments, the therapeutic compound conjugated to a sdAb as herein disclosed or encapsulated into a nanocarrier functionalized with an sdAb as herein disclosed include a nucleic acid or nucleic acid-associated molecule. In one such embodiment, the conjugated nucleic acid is a cytotoxic ribonuclease (RNase) or deoxy-ribonuclease (e.g., DNase I), an antisense nucleic acid, an inhibitory RNA molecule (e.g., a siRNA molecule) or an immunostimulatory nucleic acid (e.g., an immunostimulatory CpG motif-containing DNA molecule). In some embodiments, the antibody is conjugated to an aptamer or a ribozyme.

In some embodiments, the therapeutic compound conjugated to a sdAb (e.g., as a fusion protein) as herein disclosed or encapsulated into a nanocarrier functionalized with an sdAb as herein disclosed include a lytic peptide such as CLIP, Magainin 2, mellitin, Cecropin and PI 8.

In some embodiments, the therapeutic compound conjugated to a sdAb (e.g., as a fusion protein) as herein disclosed or encapsulated into a nanocarrier functionalized with an sdAb as herein disclosed include a cytokine, such as, e.g., IL-2, IL- 4, IL-6, IL-7, IL-10, IL-12, IL-13, IL-15, IL-18, IL-23, IL-24, IL-27, IL-28a, IL-28b, IL-29, KGF, IFNa, IFN3, IFNy, GM-CSF, CD40L, Flt3 ligand, stem cell factor, ancestim, and TNFa.

In some embodiments, the therapeutic compound conjugated to a sdAb as herein disclosed or encapsulated into a nanocarrier functionalized with an sdAb as herein disclosed include a radioisotope or to a radioisotope-containing chelate. For example, the antibody can be conjugated to a chelator linker, e.g. DOTA, DTPA or tiuxetan, which allows for the antibody to be complexed with a radioisotope. The single domain antibody may also or alternatively comprise or be conjugated to one or more radiolabeled amino acids or other radiolabeled moleculesNon- limiting examples of radioisotopes include 3H, 14C, 15N, 35S, 90Y, "Tc, 125I, 131I, 186Re, 213Bi, 225Ac and 227Th. For therapeutic purposes, a radioisotope emitting beta- or alpha-particle radiation can be used, e.g., 1311, 90Y, 211At, 212Bi, 67Cu, 186Re, 188Re, and 212Pb.

A diagnostic compound can be selected from an enzyme, a fluorophore, a NMR or MRI contrast agent, a radioisotope or a nanoparticle. For example, the diagnostic compound can be selected from the group consisting of:
- an enzyme such as horseradish peroxidase, alkaline phosphatase, glucose-6-phosphatase or beta-galactosidase;
- a fluorophore such as green fluorescent protein (GFP), blue fluorescent dyes excited at wavelengths in the ultraviolet (UV) part of the spectrum (e.g. AMCA (7-amino-4-methylcournarin-3 -acetic acid); Alexa Fluor^{®} 350), green fluorescent dyes excited by blue light (e.g. FITC, Cy2, Alexa Fluor^{®} 488), red fluorescent dyes excited by green light (e.g. rhodamines, Texas Red, Cy3, Alexa Fluor dyes 546, 564 and 594), or dyes excited with far-red light (e.g. Cy5) to be visualized with electronic detectors (CCD cameras, photomultipliers);

- a radioisotope such as 18F, nC, 13N, 150, 68Ga, 82Rb, 44Sc, 64Cu, 86Y, 89Zr, 124I, 152Tb that can be used for PET imaging or 67Ga, 81mKr, 99mTc, mIn, 123I, 125I, ,3 Xe, 201T1, 155Tb, 195mPt that can be used for SPECT / scintigraphic studies, or 14C, 3H, 35S, 3 P, 125I that can be 211 212 75 76 131 1 1 1 used for autoradiography or in situ hybridisation, or At-, Bi-, Br-, Br-, I-, In, 177Lu-, 212Pb-, 186Re-, 188Re-, 153Sm-, 0Y that can be used to label the compounds;
- a NMR or MRI contrast agent such as the paramagnetic agents gadolinium (Gd), dysprosium (Dy) and manganese (Mn), and the superparamagnetic agents based on iron oxide (such as MION, SPIO or USPIO) or iron platinium (SIPP), and X-nuclei such as 18F, 13C, 23Na, 170, 15N;
- a nanoparticle such as gold nanoparticles (B. Van de Broek et al, ACSNano, Vol. 5, No. 6, 4319-4328, 2011) or quantum dots (A. Sukhanova et al, 2012 Nanomedicine, 8 516-525).

In a preferred embodiment, said diagnostic compound is a fluorophore, more preferably Alexa Fluor^{®} 488, or a MRI contrast agent, more preferably gadolinium.

When the diagnostic agent is used for detection, it may comprise a radioactive atom for scintigraphic studies, for example 99Tc or 123I, or a spin label for nuclear magnetic resonance (NMR) imaging (also known as MRI), such as 13C, 9F, Fe, Gd, 123I, n lIn, Mn, 15N or 70.

The substance of interest according to the present disclosure may or may not permeate the mammal or human blood-brain barrier.

In some embodiments, when the compound of interest is a heterologous polypeptide, the single domain antibody of the present disclosure can be (alternatively, or in addition) fused to one or more heterologous polypeptide(s) to form a fusion protein (also named herein "fusion polypeptide" or "polypeptide"). A "fusion" or "chimeric" protein or polypeptide comprises a first amino acid sequence linked to a second amino acid sequence with which it is not naturally linked in nature. The amino acid sequences, which normally exist in separate proteins can be brought together in the fusion polypeptide. A fusion protein or polypeptide is created, for example, by chemical synthesis, or by creating and translating a polynucleotide in which the polypeptide regions are encoded in the desired relationship.

According to the present disclosure, the fusion protein can thus comprise at least one isolated humanized single domain antibody (hsbAb) according to the present disclosure that is fused either directly or via a spacer at its C-terminal end and / or at its N terminal end, notably fused at its C-terminal end to the N-terminal end of the heterologous polypeptide, and/ or at its N-terminal end to the C- terminal end of the heterologous polypeptide. As used herein, the term "directly" means that the (first or last) amino acid at the terminal end (N or C-terminal end) of the humanized single domain antibody is fused to the (first or last) amino acid at the terminal end (N or C-terminal end) of the heterologous polypeptide. In other words, in this embodiment, the last amino acid of the C-terminal end of said sdAb is directly linked by a covalent bond to the first amino acid of the N- terminal end of said heterologous polypeptide, or the first amino acid of the N-terminal end of said sdAb is directly linked by a covalent bond to the last amino acid of the C-terminal end of said heterologous polypeptide. As used herein, the term "spacer" also called "linker" refers to a sequence of at least one amino acid that links the sdAb of the present disclosure to the heterologous polypeptide. Such a spacer may be useful to prevent steric hindrances. Examples of linkers disclosed in the present disclosure have the following sequences (Gly3-Ser)4, (Gly3-Ser), Ser-Gly or (Ala- Ala- Ala).

In some embodiments the compound of interest can be one or more polypeptides comprising another or the same antigen binding domain. Notably, the compound of interest can be one or more single domain antibodies as herein disclosed or not. The resulting fusion protein, or polypeptide, that comprises two or more antigen binding domains, notably that comprises or essentially consists of two or more single domain antibodies are referred to herein as "multivalent" polypeptides or antigen binding compounds. In some embodiments, said fusion protein or polypeptide can comprise at least one single domain antibody with a first binding domain, as herein described, and at least one other binding domain (e.g. directed against the same or another epitope, antigen, target, protein or polypeptide), which is typically also a single domain antibody. "Multispecific" (fusion) polypeptide refers to a polypeptide comprising at least two different antigen binding domains (i.e. that target different epitope, antigen or target), in opposition to a polypeptide comprising similar antigen binding domains, notably comprising the same single domain antibodies ("monospecific" (fusion) polypeptide).

Thus, in some embodiments, a fusion protein as herein described may also provide at least a second antigen binding domain directed against any desired protein, polypeptide, antigen, antigenic determinant or epitope. Said binding domain can be directed against HER2, notably against the same or different HER2 epitope, or may be directed against any other antigen, polypeptide or protein.

A "bispecific" fusion protein of the present disclosure is a fusion polypeptide that comprises the single domain antibody as herein disclosed directed against a first antigen (i.e. HER2) and at least one further binding domain directed against a second HER2 epitope or antigen (i.e. different from HER2), whereas a "trispecific" polypeptide of the present disclosure is a polypeptide that comprises the single domain antibody as herein disclosed and directed against a first antigen (i.e. HER2), at least one further binding domain directed against a second HER2 epitope or antigen (i.e. different from HER2) and at least one further binding domain directed against a third HER2 epitope or antigen (i.e. different from both i.e. first and second antigen); etc.

Examples antigens other than HER2 can be selected from FGFR4, PSMA, PSCA, BCMA, CS1, GPC3, CSPG4, EGFR, fetal acetylcholine receptor gamma subunit gamma (fAChRγ), HER3, IGF1R, SLC19A1, ACVR2A, EPHB4, CA125, IL-13R, CD278, CD123, NCAM, 5T4, CD2, CD3, CD16 (FcyRIII), CD23, MART-1, L1 CAM, MUC16, ROR1 , SLAMF7, cKit, CD38, CD53, CD56, CD71, CD74, CD92, CD100, CD123, CD138, CD148, CD150, CD200, CD261, CD262, CD276, CD362, gp100, ROR1, mesothelin, CD33/IL3Ra, c-Met, Glycolipid F77, EGFRvlll, GD-2, NKp46 receptor, NY-ESO-1 TCR or MAGE A3 TCR, human telomerase reverse transcriptase (hTERT), survivin, cytochrome P450 1 B1 (CY1 B), Wilm's tumor gene 1 (WT1), livin, alphafetoprotein (AFP), carcinoembryonic antigen (CEA), mucin 16, MUC1 , p53, cyclin, an immune checkpoint target or combinations thereof.

In some embodiment, the at least one further antigen of the multispecific fusion polypeptide comprises at least an immune cell antigen such as one or more T cell antigens, one or more macrophage antigens, one or more NK cell antigens, one or more neutrophil antigens, and/or one or more eosinophil antigens, as typically exemplifier for Bispecific T-cell or NK-cell engager molecules (see notably for BiTEs^{®} Wolf E, Hofmeister R, Kufer P, Schlereth B, Baeuerle PA. "BiTEs: bispecific antibody constructs with unique anti-tumor activity". Drug Discov Today. 2005 Sep 15;10(18):1237-44. Review). Amongst others for T cell antigens, CD2 and framework sequences of T-cell receptor α and β chains can be used, notably CD2 or CD3 and most particularly the ε chain of the CD3 complex. For example, for NK cell antigens fragments from the FcyRIII and/or from the NKp46 receptor can be used.

Said multispecific polypeptide can in used immune cell redirecting immune therapies on the same principle as for CAR therapies (see for illustrative review Ellwanger K, Reusch U, Fucek I, et al. Redirected optimized cell killing (ROCK®): A highly versatile multispecific fit-for-purpose antibody platform for engaging innate immunity. MAbs. 2019;11(5):899-918).

In some embodiments, a further binding domain can be directed against a serum protein so that the half-life of the single domain antibody is increased. Typically, said serum protein is albumin.

In some embodiments, a further binding domain can be directed against a receptor on the vascular endothelium of the blood-brain barrier so that the single domain antibodies of the present disclosure would cross the blood-brain barrier. The targeted receptors include transferrin receptor, insulin receptor, IGF-I and IGF-II receptors, among others.

In some embodiments, the one or more further binding domain may comprise one or more parts, fragments or domains of conventional chain antibodies (and in particular human antibodies) and/or of heavy chain antibodies. For example, a single domain antibody as herein defined may be linked to a conventional (typically human) VH or VL optionally via a linker sequence.

In some embodiments, the polypeptides, or fusion proteins of the present disclosure can comprise a single domain antibody of the present disclosure that is linked to an immunoglobulin domain. For example, the polypeptides, or fusion proteins comprise a single domain antibody of the present disclosure that is linked to an Fc portion (such as a human Fc). Said Fc portion may be useful for increasing the half-life and even the production of the single domain antibody of the present disclosure. For example, the Fc portion can bind to serum proteins and thus increases the half-life on the single domain antibody. In some embodiments, at least one single domain antibody may also be linked to one or more (typically human) Hinge and/or CHI, and/or CH2 and/or CH3 domains, optionally via a linker sequence. For instance, a single domain antibody linked to a suitable CHI domain could for example be used - together with suitable light chains - to generate antibody fragments/structures analogous to conventional Fab fragments or F(ab')2 fragments, but in which one or (in case of an F(ab')2 fragment) both of the conventional VH domains have been replaced by a single domain antibody as herein defined. In some embodiments, one or more single domain antibodies of the present disclosure may be linked (optionally via a suitable linker or hinge region) to one or more constant domains (for example, 2 or 3 constant domains that can be used as part of/to form an Fc portion), to an Fc portion and/or to one or more antibody parts, fragments or domains that confer one or more effector functions to the polypeptide of the present disclosure and/or may confer the ability to bind to one or more Fc receptors. For example, for this purpose, and without being limited thereto, the one or more further amino acid sequences may comprise one or more CH2 and/or CH3 domains of an antibody, such as from a heavy chain antibody and more typically from a conventional human chain antibody; and/or may form and Fc region, for example from IgG (e.g. from IgGl, IgG2, IgG3 or IgG4), from IgE or from another human Ig such as IgA, IgD or IgM.

### Chimeric antigen receptors

The terms "Chimeric antigen receptor" or "CAR" or "CARs" as used herein refer to engineered receptors, which graft an antigen specificity onto cells (for example T cells such as naive T cells, central memory T cells, effector memory T cells or combination thereof) thus combining the antigen binding properties of the antigen binding domain with the lytic capacity and self renewal of T cells. CARs are also known as artificial T cell receptors, chimeric T cell receptors or chimeric immunoreceptors. The term "antigen binding domain or "antigen-specific targeting domain" as used herein refers to the region of the CAR which targets and binds to specific antigens. When a CAR is expressed in a host cell, this domain forms the extracellular domain (ectodomain).

The CAR of the present disclosure comprises a molecule of the general formula:
sdAb(n)- transmembrane domain- intracellular signaling domain,
wherein n is 1 or more.

In some embodiments, n is at least 2, for example 2, 3, 4 or 5. The sdAb(n) form the antigen binding domain and is/are located at the extracellular side when expressed in a cell.

Typically, a CAR as herein described preferably comprises one or more, notably at least two antigen binding domains (each comprising a single domain antibody), which target one or more antigen. The antigen binding domain of a CAR of the present disclosure can comprise two or at least two sdAb that are both specific for the HER2, thus providing a bivalent binding molecule. In some embodiments, the antigen binding domain comprises two or at least two VH single domain antibodies that are both specific for HER2 but bind to different epitopes. In other words, the antigen binding domain comprises a first single domain antibody that binds to a first epitope of HER2 and a second single domain antibody that binds to a second epitope of HER2. The epitopes may be overlapping. Thus, the antigen binding domain is biparatopic. In other embodiments, the antigen binding domain comprises two single domain antibodies that are both specific for HER2 and bind to the same epitope.

In preferred embodiments, the antigen binding domain comprises one single domain antibody according to the present disclosure and that is thus specific for HER2 and another antigen binding domain that is specific for another antigen, thus providing a bispecific antigen binding domain. In other words, the antigen binding domain comprises a first single domain antibody that binds to a first target consisting in HER2 and a second single domain antibody that binds to a second target. Thus, in certain embodiments, the present disclosure relates to bispecific CARs.

As used herein, the term "bispecific CAR" or "bispecifc antigen binding domain" thus refers to a polypeptide that has specificity for two targets including HER2. Accordingly, a bispecific binding molecule as described herein can selectively and specifically bind to a cell that expresses (or displays on its cell surface) HER2 and the second target.

In other embodiments, the binding molecule comprises more than two antigen-binding domains providing a multispecific binding molecule. A multispecific antigen-binding domain as described herein can thus in addition to binding HER2 bind one or more additional targets, i.e., a multispecific polypeptide can bind at least two, at least three, at least four, at least five, at least six, or more targets, wherein the multispecific polypeptide agent has at least two, at least, at least three, at least four, at least five, at least six, or more target binding sites respectively.

In some embodiments, additional antigens that can be bound by a multispecific CAR according to the present disclosure include tumor antigens. In some embodiments, the tumor antigens are associated with a hematologic malignancy or with a solid tumor. For example, a tumor antigen can be selected from the group consisting of PSMA, PSCA, BCMA, CS1, GPC3, CSPG4, EGFR, fetal acetylcholine receptor gamma subunit gamma (fAChRy), HER3, IGF1R, SLC19A1, ACVR2A, EPHB4, CA125, IL-13R, CD278, CD123, NCAM, 5T4, CD2, CD3, CD16 (FcyRIII), CD23, MART-1, L1 CAM, MUC16, ROR1 , SLAMF7, cKit, CD38, CD53, CD56, CD71, CD74, CD92, CD100, CD123, CD138, CD148, CD150, CD200, CD261, CD262, CD276, CD362, gp100, ROR1, mesothelin, CD33/IL3Ra, c-Met, Glycolipid F77, EGFRvlll, GD-2, NKp46 receptor, NY-ESO-1 TCR or MAGE A3 TCR, human telomerase reverse transcriptase (hTERT), survivin, cytochrome P450 1 B1 (CY1 B), HER2, Wilm's tumor gene 1 (WT1), livin, alphafetoprotein (AFP), carcinoembryonic antigen (CEA), mucin 16, MUC1 , p53, cyclin, an immune checkpoint target or combinations thereofHowever, a skilled person would understand that other tumor antigens are also targets within the scope of the present disclosure.

In addition to a binding domain as described in detail above, a CAR of the present disclosure further comprises a transmembrane domain. A "transmembrane domain" (TMD) as used herein refers to the region of the CAR which crosses the plasma membrane and is connected to the endoplasmic signaling domain and the antigen binding domain, in case of the latter optionally via a hinge. In one embodiment, the transmembrane domain of the CAR of the present disclosure is the transmembrane region of a transmembrane protein (for example Type I transmembrane proteins), an artificial hydrophobic sequence or a combination thereof. In some embodiments, the transmembrane domain comprises the CD3zeta domain, CD28 transmembrane domain, the CD8 alpha transmembrane domain, the DAP10 transmembrane domain or the DAP 12 transmembrane domain. Other transmembrane domains will be apparent to those of skill in the art and may be used in connection with alternate embodiments of the present disclosure.

DAP10 and DAP12 are adapters that partner with most activating NKRs expressed in NK cells and all NKRs expressed in T cells (see Chen X, Bai F, Sokol L, et al. A critical role for DAP10 and DAP12 in CD8+ T cell-mediated tissue damage in large granular lymphocyte leukemia. Blood. 2009;113(14):3226-3234).

In the immune system, DAP12 (DNAX-activation protein 12) is found in cells of the myeloid lineage, such as macrophages and granulocytes, where it associates, for instance, with the triggering receptor expressed on myeloid cell members (TREM) and MDL1 (myeloid DAP12-associating lectin 1/CLEC5A), both involved in inflammatory responses against pathogens like viruses and bacteria (for review, see Bakker A. B. et al., 1999. "Myeloid DAP12-associating lectin (MDL)-1 is a cell surface receptor involved in the activation of myeloid cells". Proc. Natl. Acad. Sci. USA 96: 9792-9796.). DAP12 possesses a single cytoplasmic immunoreceptor tyrosine-based activation motif (ITAM; D/ExxYxxL/Ix6-12YxxL/I) and signals by activating Syk protein tyrosine kinase, phosphoinositide 3-kinase (PI3K), and extracellular signal-regulated kinase (ERK/MAPK). This signaling pathway results in granule mobilization, target cell lysis, and cytokine production.

The DAP12 protein (Ref SeqGene: NG_009304.1, Uniprot ref: 043914) comprises a minimal extracellular region, mainly consisting of a cysteine residue that permits the creation of disulfide-bonded homodimers of DAP12, and which have no ligand-binding capacity. Intracellularly, DAP12 has a single ITAM, which after tyrosine phosphorylation recruits and activates notably Syk and ZAP70 in NK cells

By DAP12 it is herein intended to mean the wild-type human protein, one of its wild-type orthologs or a functional variant thereof. In any case, the functional variant comprises at least an extracellular domain, a transmembrane domain and an intracellular domain. Furthermore, a functional variant of DAP12 according to the present disclosure also comprises at least the ITAM (immunoreceptor tyrosine-based activation motif) sequence. Preferably the human wild-type DAP12 protein is used. In a well-suited embodiment, the DAP12 signal peptide (corresponding to the first 21 amino terminal amino acids including the methionine) may be replaced by another signal peptide such as the CD8 signal peptide).

DAP10 (DNAX-activation protein 10) is a type I membrane protein of 93 amino acids (Gene bank ref: human DAP10 protein: AAD47911.1). It contains a short extracellular domain, a transmembrane domain and a short cytoplasmic domain. The DAP10 cytoplasmic domain comprises an YINM signaling motif which provides co-stimulatory signaling in conjunction with the ITAM-based TCR/CD3 complex in T cells.

By full DAP10 or DAP12 it is preferably intended the human DAP10 or 12 according to the included database references and including or not the signal peptide as mentioned above. In some embodiments the CAR of the present disclosure comprises a derivative of DAP10 or DAP12 as above described comprising an extracellular domain, a transmembrane domain and an intracellular domain and having at least 90 % (typically at least 91, 92, 93, 94, 95, 96, 97, 98, 99 %) identity with DAP 10 or DAP12.

In some embodiments, the extracellular domain of the DAP10, DAP12, or of one of their functional variants is fused to the binding domain as previously defined. Typically said extracellular domain of the DAP10, DAP12, or of one of their functional variants is fused to an antibody such as a single-chain Fv antibody or a nanobody. In one alternative embodiment, said extracellular domain of the DAP10, DAP12, or of one of their functional variants is fused to a hinge fused to the binding domain.

In some embodiments the extracellular domain is fused to a hinge fused to the binding domain. A hinge may be any linker amino acid sequence comprising 2 to 50 amino acids, such as a CD8 hinge.

A CAR of the present disclosure further comprises an intracellular signaling domain. An "intracellular signaling domain", "cytoplasmic domain" or "endodomain" is the domain that transmits activation signals to T cells and directs the cell to perform its specialized function. Examples of domains that transduce the effector function signal and can be used according to the present disclosure include but are not limited to the ζ chain of the T-cell receptor complex or any of its homologs (e.g., η chain, FcsRIy and β chains, MB 1 (Iga) chain, B29 (Ig ) chain, etc.), human CD3zeta chain, CD3 polypeptides (Δ, δ and ε), syk family tyrosine kinases (Syk, ZAP 70, etc.), src family tyrosine kinases (Lck, Fyn, Lyn, etc.) and intracellular domains from other molecules involved in T-cell transduction, such as CD2, CD5, OX40, CD28, DAP 10 and DAP12. Other intracellular signaling domains will be apparent to those of skill in the art and may be used in connection with alternate embodiments of the present disclosure. In some embodiments, the intracellular domain in notably selected from the intracellular domain of DAP10, DAP12, CD28, 4-1BB or the human CD3zeta chain.

Typically, a CAR according to the present disclosure can comprise the intracellular domains of the CD3 zeta chain and of 4-1BB.

In some embodiments, the CAR can comprise additional activation domain(s) (or intracellular domain) comprising a fragment of at least 50, 60, 70, 80, 90,100, 1 10, 120, 150, or 200 amino acids of at least one additional activation domain selected from CD3-ζ chain (also shortly named ζ) and the cytoplasmic domain of a costimulatory receptors CD28, 4-1 BB (CD137), OX40 (CD134), LAG3, TRIM, HVEM, ICOS, CD27, or CD40L. In various embodiments, the CAR comprises additional activation domain(s) comprising a fragment of at least 20, 30, 40, 50, 60, 70, 80, 90,100, 1 10, 120, 150, or 200 amino acids that shares at least than 90%, preferably more than 95%, more preferably more than 99% identity with the amino acid sequence of the additional activation domain above mentioned.

In some embodiments, a CAR of the present disclosure further comprises one or more co- stimulatory domains to enhance CAR-T cell activity after antigen specific engagement. Inclusion of this domain in the CAR of the present disclosure enhances the proliferation, survival and/or development of memory cells. The co-stimulatory domain is located intracellular^. The co-stimulatory domain is a functional signaling domain obtained from a protein selected form the following group: CD3zeta, CD28, CD137 (4-IBB), CD134 (OX40), Dap10, CD27, CD2, CD5, ICAM-1, LFA-1 (CD1 la/CD18), Lck, TNFR-I, TNFR-II, Fas, CD30, CD40, LAG3, TRIM, HVEM, ICOS, CD40L or combinations thereof. Other co-stimulatory domains (e.g., from other proteins) will be apparent to those of skill in the art. Multiple co- stimulatory domains can be included in a single CAR to recruit multiple signaling pathways. In one embodiment, the co-stimulatory domain is obtained from 4-1 BB. The term "4-1 BB" refers to a member of the TNFR superfamily with an amino acid sequence provided as GenBank Acc. No. AAA62478.2, or the equivalent residues from a non- human species, e.g., rodent (e.g. mouse or rat), monkey or ape. The term "4-1 BB costimulatory domain" refers to amino acid residues 214-255 of GenBank Acc. No. AAA62478.2, or the equivalent residues from a non-human species, e.g., mouse, rodent, monkey, ape and the like.

In some embodiments of the present disclosure, the CAR only comprises DAP10, DAP12 or a variant thereof in its intracellular domain.

Example of CAR designs are notably provided in Jaspers JE, Brentjens RJ. "Development of CAR T cells designed to improve antitumor efficacy and safety" (Pharmacol Ther. 2017;178:83-91).

The results included therein showed that HER2sdAb-DAP10-z and HER2sdAb-41-z based CARs exhibit high cytotoxicity against cancer cell lines. Alternatively, while showing less cytotoxicity sdAb-DAP12 based CARs may be useful as they are expected to reduce side effects.

In some embodiments, a CAR of the present disclosure further comprises a hinge or spacer region which connects the extracellular antigen binding domain and the transmembrane domain. This hinge or spacer region can be used to achieve different lengths and flexibility of the resulting CAR. Examples of the hinge or spacer region that can be used according to the present disclosure include, but are not limited to, Fc fragments of antibodies or fragments or derivatives thereof, hinge regions of antibodies, or fragments or derivatives thereof, CH2 regions of antibodies, CH3 regions of antibodies, artificial spacer sequences, for example peptide sequences, or combinations thereof. Other hinge or spacer region will be apparent to those of skill in the art and may be used in connection with alternate embodiments of the present disclosure. In one embodiment, the hinge is an lgG4 hinge or a CD8A hinge.

In some embodiments, a CAR of the present disclosure further comprises a "linker domain" or "linker region" that connects different domains of the CAR. This domain includes an oligo- or polypeptide region from about 1 to 100 amino acids in length. Suitable linkers will be apparent to those of skill in the art and may be used in connection with alternate embodiments of the present disclosure.

In some embodiments, a CAR of the present disclosure further comprises a "leader sequence" typically in N terminal position. In one embodiment, the leader sequence is a CD8A domain.

In some embodiments the CAR further comprises a signal peptide located at the N-terminus of the polypeptide.

Suitable CAR constructs as per the present disclosure are notably disclosed in WO2019077165, or in WO2012079000A1. Advantageously according to the present disclosure, the scFv binding domain(s) as described in those patent applications is/are replaced with one or more single domain antibody and comprise(s) at least one anti-HER2 single domain antibody as herein described.

In some embodiments the signal peptide of DAP10 or DAP12 may be replaced by another signal peptide. For example, it has been noticed that the replacement of the signal peptide of DAP10 or DAP12 with the CD8 improves the CAR expression.

In some embodiments, the CAR may include a protein domain such as a SBP (streptavidin-binding peptide) domain in the C terminal region.

A CAR of the present disclosure may further include a label or a tag. For example a label that facilitates imaging, such as a fluorescent label or other tag (such as myc). This can, for example be used in methods for imaging tumor binding. The label may be conjugated to the antigen binding domain.

The CARs described herein may be synthesized as single polypeptide chains. In this embodiment, the antigen-specific targeting regions are at the N- terminus, arranged in tandem and are separated by a linker peptide.

Example of CAR designs are notably provided in Jaspers JE, Brentjens RJ. "Development of CAR T cells designed to improve antitumor efficacy and safety" (Pharmacol Ther. 2017;178:83-91). Well-suited CAR designs according to the present disclosure notably include those described by Ying, Z. et al. (A safe and potent anti-CD19 CAR T cell therapy. Nat. Med. 25, 947-953 (2019)) and by June CH, O'Connor RS, Kawalekar OU, Ghassemi S, Milone MC. CAR T cell immunotherapy for human cancer. Science. 2018 Mar 23;359(6382):1361-1365. Further suitable CAR constructs as per the present disclosure are notably disclosed in WO2019077165. Advantageously according to the present disclosure, the scFv binding domain(s) as described therein is/are replaced with one or more single domain antibody and comprise(s) at least one anti-HER2 single domain antibody as herein described.

### Nucleic acids, vectors, host cells

The present disclosure also provides isolated nucleic acids encoding a single domain antibody or a variant therefore or a CAR as previously described and nucleic acid constructs comprising thereof. A nucleic acid according to the present disclosure may be obtained by well-known methods of recombinant DNA technology and/or of chemical DNA synthesis. Also within the scope of the present disclosure, are sequences with at least 60%, 70%, 80% or 90% sequence identity thereto.

The term "nucleic acid," "polynucleotide," or "nucleic acid molecule" refers to deoxyribonucleic acid (DNA) or ribonucleic acid (RNA), or a combination of a DNA or RNA. RNA includes in vitro transcribed RNA or synthetic RNA; an mRNA sequence encoding a CAR polypeptide as described herein). The nucleic acid may further comprise a suicide gene. The construct may be in the form of a plasmid, vector, transcription or expression cassette.

The present disclosure thus also provides a recombinant expression cassette comprising a nucleic acid according to the present disclosure under the control of a transcriptional promoter allowing the regulation of the transcription of said nucleic acid in a host cell. Said nucleic acid can also be linked to appropriate control sequences allowing the regulation of its translation in a host cell.

The present disclosure also provides a recombinant vector (e.g., a recombinant expression vector) comprising a nucleic acid according to the present disclosure. Advantageously, said recombinant vector is a recombinant expression vector comprising an expression cassette according to the present disclosure.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

A vector according to the present disclosure is preferably a vector suitable for stable gene transfer and long-term gene expression into mammalian cells, such as by replication of the sequence of interest, expression of this sequence, maintaining of this sequence in extrachromosomal form, or else integration into the chromosomal material of the host. The recombinant vectors are constructed using standard recombinant DNA technology techniques and produced using conventional methods that are known in the art.

In some embodiments, a vector of the present disclosure is an integrating vector, such as an integrating viral vector, such as in particular a retrovirus or AAV vector. Preferably, the viral vector is a lentiviral vector, most preferably an integrating viral vector.

Within the context of the present disclosure, a "lentiviral vector" means a non-replicating non-pathogenic virus engineered for the delivery of genetic material into cells, and requiring lentiviral proteins (e.g., Gag, Pol, and/or Env) that are provided in trans. Indeed, the lentiviral vector lacks expression of functional Gag, Pol, and Env proteins. The lentivirus vector is advantageously a self-inactivating vector (SIN vector). The lentiviral vector comprises advantageously a central polypurine tract/DNA FLAP sequence (cPPT-FLAP), and/or insulator sequence (s) such as chicken beta-globin insulator sequence(s) to improve expression of the gene(s) of interest. The lentiviral vector is advantageously pseudotyped with another envelope protein, preferably another viral envelope protein, preferably the vesicular stomatis virus (VSV) glycoprotein. In some preferred embodiments, said lentiviral vector is a human immunodeficiency virus (HIV) vector.

Lentiviral vectors derive from lentiviruses, in particular human immunodeficiency virus (HIV-1 or HIV-2), simian immunodeficiency virus (SIV), equine infectious encephalitis virus (EIAV), caprine arthritis encephalitis virus (CAEV), bovine immunodeficiency virus (BIV) and feline immunodeficiency virus (FIV), which are modified to remove genetic determinants involved in pathogenicity and introduce new determinants useful for obtaining therapeutic effects.

The lentiviral vector may be present in the form of an RNA or DNA molecule, depending on the stage of production or development of said retroviral vectors. The lentiviral vector can be in the form of a recombinant DNA molecule, such as a plasmid, or in the form of a lentiviral vector particle (interchangeably named lentiviral particle in the context of the present disclosure), such as an RNA molecule(s) within a complex of lentiviral and other proteins.

Such vectors are based on the separation of the cis- and trans-acting sequences. In order to generate replication-defective vectors, the trans-acting sequences (e.g., gag, pol, tat, rev, and env genes) can be deleted and replaced by an expression cassette encoding a transgene.

Efficient integration and replication in non-dividing cells generally requires the presence of two c/s-acting sequences at the center of the lentiviral genome, the central polypurine tract (cPPT) and the central termination sequence (CTS). These lead to the formation of a triple-stranded DNA structure called the central DNA "flap", which acts as a signal for uncoating of the pre-integration complex at the nuclear pore and efficient importation of the expression cassette into the nucleus of non-dividing cells, such as dendritic cells. In one embodiment, the present disclosure encompasses a lentiviral vector comprising a central polypurine tract and central termination sequence referred to as cPPT/CTS sequence as described, in particular, in the European patent application EP 2 169 073.

Further sequences are usually present in cis, such as the long terminal repeats (LTRs) that are involved in integration of the vector proviral DNA sequence into a host cell genome. Vectors may be obtained by mutating the LTR sequences, for instance, in domain U3 of said LTR (AU3) (Miyoshi H et al, 1998, J Virol. 72(10):8150-7; Zufferey et al., 1998, J V/ro/ 72(12):9873-80). Preferably, the vector does not contain an enhancer. In one embodiment, the present disclosure encompasses a lentiviral vector comprising LTR sequences, preferably with a mutated U3 region (AU3) removing promoter and enhancer sequences in the 3' LTR.

The packaging sequence Ψ (psi) can also be incorporated to help the encapsidation of the polynucleotide sequence into the vector particles (Kessler et al., 2007, Leukemia, 21 (9): 1859-74; Paschen et al., 2004, Cancer Immunol Immunother 12(6): 196-203). In one embodiment, the present disclosure encompasses a lentiviral vector comprising a lentiviral packaging sequence Ψ (psi).

Further additional functional sequences, such as a transport RNA-binding site or primer binding site (PBS) or a Woodchuck PostTranscriptional Regulatory Element (WPRE), can also be advantageously included in the lentiviral vector polynucleotide sequence of the present disclosure, to obtain a more stable expression of the transgene in vivo. can also be advantageously included in the lentiviral vector polynucleotide sequence of the present disclosure, to obtain a more stable expression of the transgene in vivo. In some embodiments, the present disclosure encompasses a lentiviral vector comprising a PBS. In some embodiments, the present disclosure encompasses a lentiviral vector comprising a WPRE and/or an IRES.

Thus, in a preferred embodiment, the lentiviral vector comprises at least one cPPT/CTS sequence, one Ψ sequence, one (preferably 2) LTR sequence, and an expression cassette including a transgene under the transcriptional control of a β2ηη or class I MHC promoter.

In some embodiments of the present disclosure, a vector (i.e. a recombinant transfer vector) of the present disclosure is an expression vector comprising appropriate means for expression of the target fusion protein in a host cell.

Various promoters may be used to drive high expression of the nucleic acid sequence encoding the target fusion protein. The promoter may be a tissue-specific, ubiquitous, constitutive or inducible promoter. Preferred promoters are notably functional in T cells and/or NK cells, preferably human T cells and human NK cells. In particular, preferred promoters are able to drive high expression of the target fusion protein (notably a CAR as previously defined) from lentivectors in T cells or NK cells, preferably human T cells or NK T cells. For example, a promoter according to the present disclosure can be selected from phosphoglycerate kinase promoter (PGK), spleen focus-forming virus (SFFV) promoters, elongation factor-1 alpha (EF-1 alpha) promoter including the short form of said promoter (EFS), viral promoters such as cytomegalovirus (CMV) immediate early enhancer and promoter, retroviral 5' and 3' LTR promoters including hybrid LTR promoters, human ubiquitin promoter, MHC class I promoter, MHC class II promoter, and β2 microglobulin (β2ηη) promoter. The promoters are advantageously human promoters, i.e., promoters from human cells or human viruses such as spleen focus-forming virus (SFFV). Human ubiquitin promoter, MHC class I promoter, MHC class II promoter, and β2 microglobulin (β2ηη) promoter are more particular preferred. Preferably, the MHC class I promoter is an HLA-A2 promoter, an HLA-B7 promoter, an HLA-Cw5 promoter, an HLA-F, or an HLA-E promoter. In some embodiments the promoter is not a CMV promoter/enhancer, or is not a dectin-2 or MHCII promoter. Such promoters are well-known in the art and their sequences are available in sequence data base.

Typically, lentiviral particles refer to the extracellular infectious form of a virus composed of genetic material made from either DNA or RNA (most preferably single stranded RNA) surrounded by a protein coat, called the capsid, and in some cases an envelope of lipids that surrounds the capsid. Thus a lentiviral vector particle (or a lentiviral particle) comprises a lentiviral vector as previously defined in association with viral proteins. The vector is preferably an integrating vector.

The RNA sequences of the lentiviral particle can be obtained by transcription from a double-stranded DNA sequence inserted into a host cell genome (proviral vector DNA) or can be obtained from the transient expression of plasmid DNA (plasmid vector DNA) in a transformed host cell. Appropriate methods for designing and preparing lentiviral particles in particular for therapeutic application are well-known in the art and are for example described in Merten OW, Hebben M, Bovolenta C. Production of lentiviral vectors. Mol Ther Methods Clin Dev. 2016 Apr 13;3:16017.

Preferably the lentiviral particles have the capacity for integration. As such, they contain a functional integrase protein. Non-integrating vector particles have one or more mutations that eliminate most or all of the integrating capacity of the lentiviral vector particles. For, example, a non-integrating vector particle can contain mutation(s) in the integrase encoded by the lentiviral pol gene that cause a reduction in integrating capacity. In contrast, an integrating vector particle comprises a functional integrase protein that does not contain any mutations that eliminate most, or all of the integrating capacity of the lentiviral vector particles.

In some embodiments, the present disclosure encompasses a vector system comprising one or more vector comprising:
(a) a nucleic acid comprising a nucleic acid sequence encoding a chimeric antigen receptor as previously defined,
wherein the nucleic acids (a) and (b) are located on the same or on separated vectors.

Preferred nucleic acids (a) have been described in the prior section.

When the vector system comprises more than one vector, typically two or more vectors, said vectors are typically of the same type (e.g.: a lentiviral vector). In the following sections the vector can also be intended as "the one or more vector" or "the vector system". Preferably the present disclosure encompasses a lentiviral vector system and notably a lentiviral particle system.

According to the present disclosure, the vector can be an expression vector. The vector can be a plasmid vector.

Thus in one embodiment, the present disclosure encompasses a vector notably and expression vector, most preferably a lentiviral vector, comprising a nucleic acid encoding the CAR protein as previously defined.

The present disclosure also encompasses a viral particle system, wherein the one or more viral particle comprises a viral vector, typically an integrating viral vector, as previously defined. Preferably, the viral vector is a lentiviral vector and the viral particle is a lentiviral particle.

The present disclosure also provides a host cell containing a nucleic acid construct as herein disclosed, notably a recombinant expression cassette or a recombinant vector according to the present disclosure. The host cell is either a prokaryotic or eukaryotic host cell. The terms "host cell" refers to a cell into which exogenous nucleic acid has been introduced, including the progeny of such cells. Host cells include "transformants" and "transformed cells", which include the primary transformed cell and progeny derived therefrom without regard to the number of passages. Progeny may not be completely identical in nucleic acid content to a parent cell but may contain mutations. Mutant progeny that have the same function or biological activity as screened or selected for in the originally transformed cell are included herein.

The present disclosure also provides a method for producing in a host cell as defined above a polypeptide, consisting or comprising a single domain antibody or a CAR as previously defined, comprising the steps of:
- providing a host cell containing a nucleic acid construct, a recombinant expression cassette or a recombinant vector according to the present disclosure,
- culturing said host cell,
- and optionally purifying the single domain antibody or CAR of the present disclosure.

Methods for purifying polypeptides are well known in the art, such as chromatography (e.g., ion exchange chromatography, gel permeation chromatography and reversed phase chromatography).

The present disclosure also encompasses compositions comprising a nucleic acid construct as herein disclosed.

### Immune cells and method for obtaining thereof

The present disclosure also provides isolated cells, populations of cells, cell lines, or cell cultures, comprising a nucleic acid construct as previously described, notably vectors and more particularly a viral vector particle encoding at least one or more CAR as previously described.

In one embodiment, the cell contains the vector and/or viral vector particle integrated into the cellular genome. In one embodiment, the cell contains the vector stably expressing the CAR. In one embodiment, the cell produces lentiviral vector particles encoding the CARs.

The cells are preferably mammalian cells, particularly human cells. Particularly preferred are human non-dividing cells. Preferably, the cells are immune cells, As used herein, the term "immune cells" includes cells that are of hematopoietic origin and that play a role in the immune response. Immune cells include lymphocytes, such as B cells and T cells, natural killer cells (NK cells), myeloid cells, such as monocytes, macrophages, eosinophils, mast cells, basophils, and granulocytes.

As used herein, the term "T cell" includes cells bearing a T cell receptor (TCR), T-cells according to the present disclosure can be selected from the group consisting of inflammatory T-lymphocytes, cytotoxic T-lymphocytes, regulatory T-lymphocytes, Mucosal-Associated Invariant T cells (MAIT), Yδ T cell, tumour infiltrating lymphocyte (TILs) or helper T-lymphocytes included both type 1 and 2 helper T cells and Th17 helper cells. In another embodiment, said cell can be derived from the group consisting of CD4+ T- lymphocytes and CD8+ T-lymphocytes.

Said immune cells may originate from a healthy donor or from a subject suffering from a cancer.

Immune cells can be extracted from blood or derived from stem cells. The stem cells can be adult stem cells, embryonic stem cells, more particularly non-human stem cells, cord blood stem cells, progenitor cells, bone marrow stem cells, induced pluripotent stem cells, totipotent stem cells or hematopoietic stem cells. Representative human cells are CD34+ cells.

T-cells can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors. In certain embodiments, T-cells can be obtained from a unit of blood collected from a subject using any number of techniques known to the skilled person, such as FICOLL^{™} separation. In one embodiment, cells from the circulating blood of a subject are obtained by apheresis. In certain embodiments, T-cells are isolated from PBMCs. PBMCs may be isolated from buffy coats obtained by density gradient centrifugation of whole blood, for instance centrifugation through a LYMPHOPREP^{™} gradient, a PERCOLL^{™} gradient or a FICOLL^{™} gradient. T-cells may be isolated from PBMCs by depletion of the monocytes, for instance by using CD14 DYNABEADS^{®}. In some embodiments, red blood cells may be lysed prior to the density gradient centrifugation.

In another embodiment, said cell can be derived from a healthy donor, from a subject diagnosed with cancer, notably with Ewing sarcoma. The cell can be autologous or allogeneic.

In allogeneic immune cell therapy, immune cells are collected from healthy donors, rather than the patient. Typically, these are HLA matched to reduce the likelihood of graft vs. host disease. Alternatively, universal 'off the shelf' products that may not require HLA matching comprise modifications designed to reduce graft vs. host disease, such as disruption or removal of the TCRαβ receptor. See Graham et al., Cells. 2018 Oct; 7(10): 155 for a review. Because a single gene encodes the alpha chain (TRAC) rather than the two genes encoding the beta chain, the TRAC locus is a typical target for removing or disrupting TCRαβ receptor expression. Alternatively, inhibitors of TCRαβ signalling may be expressed, e.g. truncated forms of CD3ζ can act as a TCR inhibitory molecule. Disruption or removal of HLA class I molecules has also been employed. For example, Torikai et al., Blood. 2013;122:1341-1349 used ZFNs to knock out the HLA-A locus, while Ren et al., Clin. Cancer Res. 2017;23:2255-2266 knocked out Beta-2 microglobulin (B2M), which is required for HLA class I expression. Ren et al. simultaneously knocked out TCRαβ, B2M and the immune-checkpoint PD1. Generally, the immune cells are activated and expanded to be utilized in the adoptive cell therapy. The immune cells as herein disclosed can be expanded in vivo or ex vivo. The immune cells, in particular T-cells can be activated and expanded generally using methods known in the art. Generally the T-cells are expanded by contact with a surface having attached thereto an agent that stimulates a CD3/TCR complex associated signal and a ligand that stimulates a co-stimulatory molecule on the surface of the T cells.

Typically, the immune cell is modified to express chimeric antigen receptor as herein disclosed. Expression of multiple tumor-specific targets may reduce the chance of antigen escape by mutating or reducing expression of the target antigen. As previously described the CARs of the present disclosure may be multispecific CARs (i.e. directed against more than one antigen, that is directed against HER2 and at least another antigen). In addition, or alternatively, an immune cell as herein described may express one or more CAR(s) as herein defined and at least another CAR targeting one or more other antigen(s).

Methods by which immune cells can be genetically modified to express a recombinant antigen receptor are well known in the art. A nucleic acid molecule encoding the antigen receptor may be introduced into the cell in the form of e.g. a vector, or any other suitable nucleic acid construct. Vectors, and their required components, are well known in the art. Nucleic acid molecules encoding antigen receptors can be generated using any method known in the art, e.g. molecular cloning using PCR. Antigen receptor sequences can be modified using commonly-used methods, such as site-directed mutagenesis.

In another aspect, the present disclosure relates to an *ex vivo* method for generating a population of cells for use in adaptive immunotherapy comprising transforming said cell with a CAR as herein described.

### Compositions and kits of the present disclosure

The present disclosure also encompasses pharmaceutical compositions comprising one or more anti-HER2 single domain antibody(ies), CAR(s), nucleic acid construct encoding thereof and/or one or more isolated cell(s) or cell population(s) comprising a CAR as herein disclosed, alone or in combination with at least one other agent, such as a stabilizing compound, which may be administered in any sterile, biocompatible pharmaceutical carrier and optionally formulated with formulated with sterile pharmaceutically acceptable buffer(s), diluent(s), and/or excipient(s). Pharmaceutically acceptable carriers typically enhance or stabilize the composition, and/or can be used to facilitate preparation of the composition. Pharmaceutically acceptable carriers include solvents, dispersion media, coatings, antibacterial and antifungal agents, isotonic and absorption delaying agents, and the like that are physiologically compatible, and in some embodiments, pharmaceutically inert. In some embodiment, a pharmaceutical composition according to the present disclosure comprises an anti- HER2 single domain antibody as herein disclosure linked to drug nanocarrier as previously disclosed. Typically said drug nanocarriers, dunctionalized with an anti-HER2 sdAb as herein disclosed are encapsulated a therapeutic (such as a cytotoxic) or a diagnostic compound. In particular embodiments, said drug nanocarriers are liposomes.

Administration of a pharmaceutical composition comprising sdAbs as herein disclosed can be accomplished orally or parenterally. Methods of parenteral delivery include topical, intra-arterial (directly to the tumor), intramuscular, spinal, subcutaneous, intramedullary, intrathecal, intraventricular, intravenous, intraperitoneal, or intranasal administration.

The genetically modified cells or pharmaceutical composition of the present disclosure can be administered by any convenient route, including parenteral administration. Parenteral administration includes, for example, intravenous, intramuscular, intraarterial, intraperitoneal, intranasal, rectal, intravesical, intradermal, topical or subcutaneous administration. Compositions can take the form of one or more dosage units.

Thus, in addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Ed. Maack Publishing Co, Easton, Pa.).

Depending on the route of administration, the single domain antibody or variant thereof, may be coated in a material to protect the compound from the action of acids and other natural conditions that may inactivate the compound.

The composition is typically sterile and preferably fluid. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions and the like, for ingestion by the patient.

Pharmaceutical preparations for oral use can be obtained through combination of active compounds with solid excipient, optionally grinding a resulting mixture, and processing the mixture of granules, after adding suitable auxilliaries, if desired, to obtain tablets or dragee cores. Suitable excipients are carbohydrate or protein fillers such as sugars, including lactose, sucrose, mannitol, or sorbitol; starch from corn, wheat, rice, potato, or other plants; cellulose such as methyl, cellulose, hydroxypropylmethylcellulose, or sodium carboxymethylcellulose; and gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinylpyrrolidone, carbopol gel, polyethylene glycol and/or titanium dioxide, lacquer solutions, and suitable organic solvents or solvent mixtures. Dyestuffs or pigments may be added to the tablets or dragee coatings for product identification or to characterize the quantity of active compound, ie. dosage.

Pharmaceutical preparations that can be used orally include push-fit capsules made of gelatin, as well as soft, sealed capsules made of gelatin and a coating such as glycerol or sorbitol. Push-fit capsules can contain active ingredients mixed with a filler or binders such as lactose or starches, lubricants such as talc or magnesium stearate, and optionally, stabilizers. In soft capsules, the active compounds may be dissolved or suspended in suitable liquids, such as fatty oils, liquid paraffin, or liquid polyethylene glycol with or without stabilizers.

Pharmaceutical formulations for parenteral administration include aqueous solutions of active compounds. For injection, the pharmaceutical compositions of the present disclosure may be formulated in aqueous solutions, preferably in physiologically compatible buffers such as Hank's solution, Ringer's solution, or physiologically buffered saline. Aqueous injection suspensions may contain substances that increase viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

Pharmaceutical compositions of the disclosure can be prepared in accordance with methods well known and routinely practiced in the art. See. e.g., Remington: The Science and Practice of Pharmacy, Mack Publishing Co., 20th ed., 2000; and Sustained and Controlled Release Drug Delivery Systems, J R. Robinson, ed., Marcel Dekker, Inc., New York, 1978. Pharmaceutical compositions are preferably manufactured under GMP conditions.

The amount of the pharmaceutical composition of the present disclosure that is effective/active in the treatment of a particular disorder or condition will depend on the nature of the disorder or condition, and can be determined by standard clinical techniques. In addition, in vitro or in vivo assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The compositions as herein disclosed comprise an effective amount of a binding molecule of the present disclosure (e.g. a single domain antibody or variant thereof or a chimeric antigen receptor) such that a suitable dosage will be obtained. The correct dosage of the compounds will vary according to the particular formulation, the mode of application, and its particular site, host and the disease being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

Typically, this amount is at least about 0.01 % of a binding molecule of the present disclosure by weight of the composition. Preferred compositions of the present disclosure are prepared so that a parenteral dosage unit contains from about 0.01 % to about 2% by weight of the binding molecule of the present disclosure.

For intravenous administration, the composition can comprise from about typically about 0.1 mg/kg to about 250 mg/kg of the animal's body weight, preferably, between about 0.1 mg/kg and about 20 mg/kg of the animal's body weight, and more preferably about 1 mg/kg to about 10 mg/kg of the animal's body weight.

The present compositions can take the form of suitable carriers, such aerosols, sprays, suspensions, or any other form suitable for use. Other examples of suitable pharmaceutical carriers are described in "Remington's Pharmaceutical Sciences" by E. W. Martin.

The pharmaceutical compositions as herein disclosed can be co-administered with other therapeutics, for example anti-cancer agents.

### Therapeutic uses

The present disclosure also relates to an anti-HER2 single domain antibody or variant thereof as herein described, a CAR directed against HER2 or variant thereof as herein described, a nucleic acid encoding said anti- HER2 single domain antibody or CAR, or to a cell, line or cell population comprising a CAR as described herein for use in therapy, in particular, for use in the treatment of cancer. The present disclosure also relates to an anti-HER2 single domain antibody or variant thereof as herein described, a CAR directed against HER2 or variant thereof as herein described, a nucleic acid encoding said anti-HER2 single domain antibody or CAR, or to a cell, line or cell population comprising said CAR as described herein in the manufacture of a medicament, notably for the treatment of cancer.

The present disclosure also encompasses methods for the prevention and/or treatment of cancer, comprising administering to a subject to an anti-HER2 single domain antibody or variant thereof as herein described, a CAR directed against HER2 or variant thereof as herein described, a nucleic acid encoding said anti-HER2 single domain antibody or CAR, or a cell, line or to a cell population comprising a CAR as described herein, said method comprising administering, to a subject in need thereof, a pharmaceutically active amount of an anti-HER2 single domain antibody or variant thereof, a CAR, a cell, line or cell population comprising a CAR as described herein and/or of a pharmaceutical composition of the present disclosure. The method may additionally comprise the step of identifying a subject who has cancer.

The present disclosure also include the use of one or more of to the anti-HER2 single domain antibodies or variants thereof, CARs directed against HER2 or variants thereof, nucleic acids encoding said anti-HER2 single domain antibodies or CARs, cell lines or cell population comprising a CAR as described herein in targeted immune therapy. For example sdAbs of the present disclosure and in particular variants thereof in the form of multispecific polypeptides further targeting an immune cell antigen, and CAR expressing immune cells (notably CAR T cells) may be used in immune cell redirecting immune therapies.

In another aspect, the present disclosure relates to a method for stimulating a T cell-mediated immune response to a target cell population or tissue in a subject, the method comprising administering to a subject an effective amount of a cell or cell population that expresses a CAR directed against HER2 as herein described.

In another aspect, the present disclosure relates to a method of providing an anti-tumor immunity in a subject, the method comprising administering to the mammal an effective amount of a cell or cell population genetically modified to express a CAR directed against HER2 as herein described, thereby providing an anti- tumor immunity in the subject.

The present disclosure also relates to an anti-HER2 single domain antibody (including variants thereof), a CAR directed against HER2 as herein described, or a nucleic acid construct encoding said humanized anti-HER2 SdAb or CAR, or to an immune cell expressing said CAR, as previously defined, for use in adoptive cell or CAR-T cell therapy in a subject. Typically, the immune cell for use in the method of the present disclosure is a redirected T-cell, e.g. a redirected CD8+ and/ or CD4+ T-cell.

In some embodiments, anti-HER2 single domain antibodies (including variants thereof), and CARs directed against HER2 as herein described, as well as nucleic acid constructs encoding them and cells comprising such CARs are useful for inhibiting tumor growth, inducing differentiation, reducing tumor volume, and/or reducing the tumorigenicity of a tumor. The methods of use can be in vitro, ex vivo, or in vivo methods.

In specific embodiments of the medical uses as described herein, the anti-HER2 sdAb is linked as previously described to a drug nanocarrier, such as a liposome. Said drug nanocarrier, notably such liposome, functionalized with one or more anti-HER2 sdAbs as herein described typically encapsulates a therapeutic compound (such as a cytotoxic compound) or a diagnostic compound.

In certain aspects, the subject is a human, notably a pediatric patient. In certain aspects, the subject has a tumor or has had a tumor removed. The subject can also be at risk of developing a cancer.

The cancer can be a solid cancer or a liquid tumor. Cancers that may treated by methods, uses and compositions described herein include, but are not limited to, cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma (RMS); embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

More specific cancers which can be treated and/or prevented according to the present disclosure include HER2-mediated cancers. Typically, HER2-mediated cancers are cancers wherein HER2 is expressed or overexpressed. Typical cancers wherein HER2 is expressed and/or overexpressed include hepatocellular carcinoma (HCC), breast cancer, oropharyngeal squamous cell carcinoma, oral squamous cell carcinoma, pancreatic carcinomas and derived cell lines and rhabdomyosarcoma (RMS).

In some embodiments, cancer treatment, and/or adoptive cell cancer therapy as above described are administered in combination with additional cancer therapies. , In some embodiments, cancer treatment and/or adoptive cell cancer therapy as above described are administered in combination with targeted therapy, immunotherapy such as immune checkpoint therapy and immune checkpoint inhibitor, co-stimulatory antibodies, chemotherapy and/or radiotherapy.

Immune checkpoint therapy such as checkpoint inhibitors include, but are not limited to programmed death-1 (PD-1) inhibitors, programmed death ligand-1 (PD-L1) inhibitors, programmed death ligand-2 (PD-L2) inhibitors, lymphocyte-activation gene 3 (LAG3) inhibitors, T-cell immunoglobulin and mucin-domain containing protein 3 (TIM-3) inhibitors, T cell immunoreceptor with Ig and ITIM domains (TIGIT) inhibitors, B- and T-lymphocyte attenuator (BTLA) inhibitors, V-domain Ig suppressor of T-cell activation (VISTA) inhibitors, cytotoxic T-lymphocyte-associated protein 4 (CTLA4) inhibitors, Indoleamine 2,3-dioxygenase (IDO) inhibitors, killer immunoglobulin-like receptors (KIR) inhibitors, KIR2L3 inhibitors, KIR3DL2 inhibitors and carcinoembryonic antigen-related cell adhesion molecule 1 (CEACAM-1) inhibitors. In particular, checkpoint inhibitors include antibodies anti-PD1, anti-PD-L1, anti-CTLA-4, anti-TIM-3, anti-LAG3. Co-stimulatory antibodies deliver positive signals through immune-regulatory receptors including but not limited to ICOS, CD137, CD27, OX-40 and GITR.

Example of anti-PD1 antibodies include, but are not limited to, nivolumab, cemiplimab (REGN2810 or REGN-2810), tislelizumab (BGB-A317), tislelizumab, spartalizumab (PDR001 or PDR-001), ABBV-181, JNJ-63723283, BI 754091, MAG012, TSR-042, AGEN2034, pidilizumab, nivolumab (ONO-4538, BMS-936558, MDX1106, GTPL7335 or Opdivo), pembrolizumab (MK-3475, MK03475, lambrolizumab, SCH-900475 or Keytruda) and antibodies described in International patent applications WO2004004771, WO2004056875, WO2006121168, WO2008156712, WO2009014708, WO2009114335, WO2013043569 and WO2014047350.

Example of anti-PD-L1 antibodies include, but are not limited to, LY3300054, atezolizumab, durvalumab and avelumab.

Example of anti-CTLA-4 antibodies include, but are not limited to, ipilimumab (see, e.g., US patents US6,984,720 and US8,017,114), tremelimumab (see, e.g., US patents US7,109,003 and US8,143,379), single chain anti-CTLA4 antibodies (see, e.g., International patent applications WO1997020574 and WO2007123737) and antibodies described in US patent US8,491,895.

Example of anti-VISTA antibodies are described in US patent application US20130177557.

Example of inhibitors of the LAG3 receptor are described in US patent US5,773,578.

Example of KIR inhibitor is IPH4102 targeting KIR3DL2.

As used herein, the term "chemotherapy" has its general meaning in the art and refers to the treatment that consists in administering to the patient a chemotherapeutic agent. A chemotherapeutic entity as used herein refers to an entity which is destructive to a cell, that is the entity reduces the viability of the cell. The chemotherapeutic entity may be a cytotoxic drug. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; methylhydrazine derivatives including N-methylhydrazine (MIH) and procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; anthracyclines, nitrosoureas, antimetabolites, epipodophylotoxins, enzymes such as L-asparaginase; anthracenediones; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide; biological response modifiers such as IFNa, IL-2, G-CSF and GM-CSF; and pharmaceutically acceptable salts, acids or derivatives of any of the above.

Suitable examples of radiation therapies include, but are not limited to external beam radiotherapy (such as superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT), 3-dimensional conformal radiation therapy (3D-CRT) and the like); brachytherapy; unsealed source radiotherapy; tomotherapy; and the like. Gamma rays are another form of photons used in radiotherapy. Gamma rays are produced spontaneously as certain elements (such as radium, uranium, and cobalt 60) release radiation as they decompose, or decay. In some embodiments, radiotherapy may be proton radiotherapy or proton minibeam radiation therapy. Proton radiotherapy is an ultra-precise form of radiotherapy that uses proton beams (Prezado Y, Jouvion G, Guardiola C, Gonzalez W, Juchaux M, Bergs J, Nauraye C, Labiod D, De Marzi L, Pouzoulet F, Patriarca A, Dendale R. Tumor Control in RG2 Glioma-Bearing Rats: A Comparison Between Proton Minibeam Therapy and Standard Proton Therapy. Int J Radiat Oncol Biol Phys. 2019 Jun 1;104(2):266-271. doi: 10.1016/j.ijrobp.2019.01.080; Prezado Y, Jouvion G, Patriarca A, Nauraye C, Guardiola C, Juchaux M, Lamirault C, Labiod D, Jourdain L, Sebrie C, Dendale R, Gonzalez W, Pouzoulet F. Proton minibeam radiation therapy widens the therapeutic index for high-grade gliomas. Sci Rep. 2018 Nov 7;8(1):16479. doi: 10.1038/s41598-018-34796-8). Radiotherapy may also be FLASH radiotherapy (FLASH-RT) or FLASH proton irradiation. FLASH radiotherapy involves the ultra-fast delivery of radiation treatment at dose rates several orders of magnitude greater than those currently in routine clinical practice (ultra-high dose rate) (Favaudon V, Fouillade C, Vozenin MC. The radiotherapy FLASH to save healthy tissues. Med Sci (Paris) 2015 ; 31 : 121-123. DOI: 10.1051/medsci/20153102002); Patriarca A., Fouillade C. M., Martin F., Pouzoulet F., Nauraye C., et al. Experimental set-up for FLASH proton irradiation of small animals using a clinical system. Int J Radiat Oncol Biol Phys, 102 (2018), pp. 619-626. doi: 10.1016/j.ijrobp.2018.06.403. Epub 2018 Jul 11).

"In combination" may refer to administration of the additional therapy before, at the same time as or after administration of the T cell composition according to the present disclosure.

In addition, or as an alternative to the combination with checkpoint blockade, the T cell composition of the present disclosure may also be genetically modified to render them resistant to immune-checkpoints using gene-editing technologies including but not limited to TALEN and Crispr/Cas. Such methods are known in the art, see e.g. US20140120622. Gene editing technologies may be used to prevent the expression of immune checkpoints expressed by T cells (see the above listed checkpoint inhibitors) and more particularly but not limited to PD-1, Lag-3, Tim-3, TIGIT, BTLA CTLA-4 and combinations of these. The T cell as discussed here may be modified by any of these methods.

The T cell according to the present disclosure may also be genetically modified to express molecules increasing homing into tumors and or to deliver inflammatory mediators into the tumor microenvironment, including but not limited to cytokines, soluble immune-regulatory receptors and/or ligands.

Having thus described different embodiments of the present disclosure, it should be noted by those skilled in the art that the disclosures herein are exemplary only and that various other alternatives, adaptations, and modifications may be made within the scope of the present disclosure. Accordingly, the present disclosure is not limited to the specific embodiments as illustrated herein.

The present invention also relates to the use of a modified immune cell according to the invention, as a medicament.

The present invention also relates to a pharmaceutical composition comprising a modified immune cell according to the invention, and a pharmaceutical acceptable carrier.

The present invention also relates to the use of the modified immune cell according to the invention, or of the pharmaceutical composition described above, in the treatment of cancer.

Preferably, the modified cells are obtained from a blood sample of the treated patient (treated donor). Preferably, they are modified and reinjected to the patient (donor), i.e. they are autologous.

By "autologous", the present invention means that the modified immune cells, the modified myeloid cells or the therapeutic composition comprising the modified immune cells, the modified myeloid cells are used as a treatment for the patient from which the immune cells or myeloid cells are originated.

### Molecular Imaging and diagnostic tool

The nanobodies as herein disclosed are of high interest in molecular imaging and diagnostic (both *in vitro* and *in vivo*)*,* typically to target imaging agents such as radionuclides to the cell of interest *in vivo.* Thus nanobody-targeted imaging can be used according to the present invention for a variety of purposes including the diagnostic of diseases, the monitoring of disease progression and the prediction of response to a specific therapeutic agent in particular to an anti-HER2 agent such as an anti-HER2 sdAb as herein disclosed.

Nanobodies can aid in early diagnosis and cancer prevention by detecting or defining biomarkers. Nanobodies can improve current mAb-based diagnostic techniques due to their high specificity. Furthermore, their high stability under extremes of temperature, pH, or ionic strength, ensures that the application still can occur under harsh conditions.

The small size of nanobodies is highly advantageous especially in the field of molecular imaging as it enables rapid tumor accumulation and homogenous distribution as well as efficient blood clearance, contributing to high tumor-to-background ratios.

Nanobodies as herein described, for use in molecular imaging and/or as a diagnostic tool can be easily conjugated to several kinds of imaging agents and their high specificity renders their use relatively safe.

In some embodiments, the nanobodies as herein disclosed can be conjugated to radionuclides for use in radio-imaging. Single-photon emission computed tomography (SPECT) is based on γ-rays and sdAb of the present disclosure can thus linked to longer-lived radionuclides such as 99mTc, 177Lu, 123I, 125I and 111In. On the other hand, shorter-lived radionuclides such as 68Ga, 124I or 89Zr, 64Cu, 18F, or 150 can be used for positron emission tomography (PET) purposes. Scintigraphy may use the same radionuclides as SPECT.

Other imaging agents usable is nanobody-targeted based imaging as per the present disclosure includes absorbing small-molecule dyes, metallic nanoparticles (photoacoustic imaging, PAI), small synthetic fluorescent probes, which popular imaging agent includes near-infrared fluorophores such as IRDye 800CW, Ag2S quantum dots and the FDA-approved indocyanine green, or fluorescent protein-expressing genes (fluorescence molecular tomography), luciferase expressing genes (bioluminescence imaging), Superparamagnetic iron oxide (SPIO), gadolinium-DTPA (magnetic resonance imaging), Nanoparticle-based contrast agents (computed tomography).

sdAbs are typically highly relevant in molecular imaging strategy due to their small size and fast clearance. They also possess high chemical and temperature resistance due to their small size and less complex 3D structure. This is thus favorable for molecular imaging procedures as well as for conjugation chemistry (production).

Anti-HER2 sdAbs as per the present disclosure can also be used in cell-based ELISA assays. To perform sandwich ELISA, both a capturing and detecting nanobody are used, preferably targeting different epitopes on the antigen.

In some embodiments of the present disclosure, the anti-HER2 single domain antibodies as herein described are thus useful for detecting the presence of HER2 in a biological sample. The term "detecting" as used herein encompasses quantitative or qualitative detection. In certain aspects, a biological sample comprises one or more cell(s) or tissue(s). In certain aspects, such tissues include normal and/or cancerous tissues that express HER2, notably that express HER2 at higher levels relative to other tissues or similar tissue from a control subject or from a control population of subjects.

Also included is a method of diagnosing a disorder associated with an increased expression of HER2, typically HER2-associated cancers or tumors. In certain aspects, the method comprises:
- contacting a test cell with an anti-HER2 single domain antibody of the present disclosure;
- determining the level of expression (either quantitatively or qualitatively) of HER2 on the test cell by detecting binding of said humanized anti-HER2 sdAb to HER; and
- comparing the level of expression of HER2 in the test cell with the level of expression of HER2 in a control cell (e.g., a normal cell of the same tissue origin as the test cell or a cell that expresses HER2 at levels comparable to such a normal cell), wherein a higher level of expression of HER2 on the test cell as compared to the control cell indicates the presence of a disorder associated with increased expression of HER2. In certain aspects, the test cell is obtained from an individual suspected of having a disorder associated with increased expression of HER2. In certain aspects, the disorder is a cell proliferative disorder, such as a cancer or a tumor.

In certain aspects, a method of diagnosis or detection, such as those described above, comprises detecting binding of an anti-HER2 single domain antibody expressed on the surface of a cell or in a membrane preparation obtained from a cell expressing HER2 on its surface. An exemplary assay for detecting binding of an humanized anti-HER2 sdAb to HER2 expressed on the surface of a cell is a "FACS" assay.

Certain other methods can be used to detect binding of humanized anti-HER2 sdAb as herein disclosed to HER2. Such methods include, but are not limited to, antigen-binding assays that are well known in the art, such as western blots, radioimmunoassays, ELISA (enzyme linked immunosorbent assay), "sandwich" immunoassays, immunoprecipitation assays, fluorescent immunoassays, protein A immunoassays, and immunohistochemistry (IHC). Advantageously in these embodiments humanized anti-HER2 sdAbs as herein disclosed are linked to a diagnostic compound, in particular a detectable label, as previously described.

In some embodiments of the methods as herein described, the anti-HER2 sdAb as herein disclosed is linked to a drug nanocarrier, such as a liposome. Typically, said drug nanocarrier functionalized with anti-HER2 sdAb as herein disclosed encapsulates a diagnostic compound.

The invention will be further illustrated by the following examples. However, these examples should not be interpreted in any way as limiting the scope of the present invention.

### EXAMPLE:

### Fully humanized anti-HER2 single domain antibody (sdAb) and anti-HER2 sdAb CAR-T

Targeting tumor-specific epitope is essential for various diagnostics and therapeutic approaches. Chimeric antigen receptor (CAR) technology has revolutionized the field of immunotherapy, demonstrating high efficient in the fight against hematologic malignancies. These results leaded to Food and Drug Administration (FDA) and European Medicines Agency (EMA) approval of two CAR-T cell therapies using an scFv-CD19 against malignant B cells, the Klymriah and Yescarta. However, until now, no CAR-T cells against solid tumor has shown such efficiency. Therefore, it remains crucial the development and optimization of CAR-T cells against solid tumor but also with limited side effects that could compromise the patient's life. CARs are usually composed by an antibody-derived fragment, usually a single chain variable fragment (scFv), fused to a transmembrane domain and co-stimulatory motives required for immune cells survival, persistence and effector activity.

The inventors have been working on engineering CAR cell, namely in the development of a system to control the transport of the CAR to the surface upon a specific stimulus, referred to as CellTune (for the generic system) and as CARTune (for CAR-T cell therapy). The inventors have also been exploring other co-stimulatory motives that have been described to be sufficient for T cells activation, these include DAP10 and DAP12 co-receptors. The inventors demonstrated that those motives once adapted to a scFv-CD19 CAR are capable to induce cytotoxicity of the T cell against tumor B cell using in vitro assays. Interestingly, these different scaffolds differently tune the activity of the CAR which is one of important parameters to reduce its toxic effects. The inventors applied these technologies to other antibody-derived fragments targeting other tumor antigens. These includes the nanobody selected against HER2 positive cell lines that were selected from a humanized nanobodies (or vHH) library. The CAR containing vHH against HER2 positive cell line will be used to target solid tumors, such as breast cancer, and the different scaffolds of the CAR will allow us to select the best balance between high efficiency and reduced toxic effects.

Single domain antibodies have been described to induce lower side effects when compared with the scFv. The inventors selected a new binder, a fully human VH single domain antibody for therapeutic applications. Therefore, by selecting a vH and a CAR scaffold that induce killing but lower toxic effects, the inventors aim to bring these technology to clinic trials.

The present invention aims at targeting solid tumors expressing HER2, using a VH selected against HER2 adapted to CAR scaffold. The inventors confirmed *in vitro* its efficacy as a CAR with the current scaffold used in clinics (41BB-CD3zeta).

The inventors adapt this CAR to the CARTune system (WO2016/012623; CAR RUSH), allowing the modulation of the T cell effector activity and possible side effects. This should be a good alternative to reduce known side effects associated with HER2 CAR therapy (Morgan, Yang *et al.,* 2010).

The inventors also adapt this CAR to a CAR "split" format to reduce side effect because of off-targets (Xin He, Zijie Feng, Jian Ma, Sunbin Ling, Yan Cao, Buddha Gurung, Yuan Wu, Bryson W. Katona, Kienan P. O'Dwyer, Don L. Siegel, Carl H. June, Xianxin Hua; Bispecific and split CAR T cells targeting CD 13 and TIM3 eradicate acute myeloid leukemia. Blood 2020; 135 (10): 713-723. doi: https://doi.org/10.1182/blood.2019002779).

Single domain antibodies (VHH or VH) can be a good alternative to the antibody derived fragment of the CAR, instead of the scFv which has several drawbacks that compromise the therapeutic application of CAR. These include large size, need of structure stabilization by disulfide bonds and possible CAR clustering due to ligand-independent oligomerization of scFv. This can lead to CAR tonic signalling with non-specific activation that can induce non-specific cell killing, T cell overactivation, premature T cell exhaustion and/or inhibition of CAR-T cell activity (Long, Haso *et al.* 2015).

These technologies can be used to target solid tumor expressing HER2 which is of interest for cell-based therapies. This technology can be further used in combination to the CellTune and more specifically CARTune technology. The sdAB of the invention may also be used in combination with the 4th generation CAR or similar. In these generation, T cells target specifically the tumor thought the CAR recognition and in simultaneous induces the production and/or secretion of therapeutic relevant checkpoint inhibitors, cytokines or other dominant active or dominant negative proteins.

### Results

From inventors humanized sdAb libraries, a phage display subtraction selection system was developed to identify sdAbs that selectively detect the surface of breast tumor cells. The SKBR3 target cell line was used because it overexpresses the HER2 protein on its surface, while the MCF10A cell line, derived from non-cancerous and HER2-negative mammary gland cells, was used to pre-adsorb the libraries. After 3 successive cycles of selection on these cell lines, sdAb clones were analyzed by flow cytometry (FACS) for their binding capacity on SKBR3 cells and MCF10A cells.

A human VH anti-HER2 was selected for further experiments. The humanized anti-HER2 sdAb of the invention shown specific recognition on SKBR3 cells and not on MCF10A cells in FACS experiment (data not shown).

The anti-HER2 sdAb was afterward validated by ELISA on Her2 recombinant ectodomain fused to a Rabbit Fc domain (data not shown).

The anti-HER2 sdAb of the invention was also validated for use in CAR-T format. The model of CAR chosen is the one used to treat diffuse lymphomas (Linguanti F, Abenavoli EM, Berti V, Lopci E. Metabolic Imaging in B-Cell Lymphomas during CAR-T Cell Therapy. Cancers (Basel). 2022 Sep 27;14(19):4700. doi: 10.3390/cancers14194700. PMID: 36230629; PMCID: PMC9562671). This anti-lymphoma CAR-T therapy called "second generation" comprises the transmembrane domain of CD8, a costimulation domain from 4-1BB and the CD3z signaling domain. In the case of Kymriah^{®} targeting B lymphocytes, the binding module is an anti-CD19 scFv. In the construction of the invention, the anti-CD19 scFv was replaced by the anti-Her2 sdAb of the invention.

The inventors developed a reporter cell system allowing to identify CAR-T cell activity. It is based on a Jurkat-derived clone bearing a NFAT-YFP gene reporter activated by TCR clustering (Blanchard N. et al, The Journal of Immunology, 2004).

The anti-Her2 sdAb of the invention showed specific activation that can be detected by FACS. Again, CAR including the anti-Her2 sdAb of the invention shows a better T cell activation than CAR including anti-Her2 VHH.

### In vivo experiments using the anti-HER2 sdAb CAR-T and comparison with the scFv CAR-T of reference in a Her2 mouse model

The inventors carry out *in vivo* experiments to confirm CAR-T activity in a grafted HER2 tumors model. The best timing for the injection of CAR-T cells after tumor grafting, number of cells to inject and time of incubation have already been determined.

The inventors also compare *in vivo* the activity of the sdAb of the invention in preclinical models with the CAR-T derived from Trastuzumab. This study allows to obtain comparison data with a reference treatment and to demonstrate the benefits of the technology and the potential of the CAR-T product armed with sdAb of the invention. The *in vivo* validated anti-Her2 sdAb may also be used for the development of further therapeutic product in a CAR-T split format.

### Results

Detailed Experimental Work Plan includes:
- Obtaining batches of CAR-containing lentivirus
- Infection of T cells with lentivirus
- *In vivo* experiments to measure tumor size

The efficacy of CAR-T cells was then validated *in vivo,* and experiments were conducted in two stages to obtain T lymphocytes from two different donors and minimize donor-dependent risk factors.

For each experiment, the inventors had:
- 5 mice per group.
- Mice grafted intraperitoneally with an ovarian tumor cell line overexpressing Her2 receptor.

### Experimental design:

J-3: Injection of SK-OV-1 10⁶ cells to the mice.
J1: Injection of the CAR-T 3. 10⁶ ells to the mice.

Then PET-scan every week.
- 5 untreated mice, injected with PBS.
- 5 mice treated with CAR-T cells armed with the clinical anti-CD19 scFv (negative control).
- 5 mice treated with CAR-T cells armed with the VHH anti-Her2.
- 5 mice treated with CAR-T cells armed with the anti-Her2 sdAb of the invention from the Gimli library.

### 1. In vitro validation of sdAbs in CAR-T format:

T lymphocytes from 2 healthy donors from the EFS were purified. These lymphocytes were then infected at a MOI=10 (i.e., 10 viral particles per T lymphocyte) with different CAR-T constructs. The expression of CAR on the surface of T lymphocytes was confirmed by flow cytometry using an antibody recognizing an AlfaTag between the plasma membrane and the antibody, proving its presence on the surface of the T cell (data not shown).

The inventors observed that regardless of the donor, the CAR armed with the scFv, VHH or anti-HER2 sdAb of the invention was expressed on the surface of T lymphocytes (data not shown).

A cytotoxicity test against SK-OV-3 Luc cells overexpressing the HER2 receptor was then performed. The target tumor cells were incubated for 48 hours with increasing numbers of CAR-T cells (effectors). Effector/target (E:T) ratios of 1/1, 2/1, 4/1, or 8/1 were used. The lysis of tumor cells was measured by the release of luciferase into the culture medium. Wild-type lymphocytes from the donor were also incubated with the target cells.

The inventors observed that for both donors, all three anti-Her2 CAR-T cells achieved a very high percentage of killed tumor cells.

### 2. In vivo validation of sdAbs in CAR-T format:

T lymphocytes from new donors were infected with the three different anti-Her2 CARs for further *in vivo* experiments. The expression of CAR constructs on the surface of T lymphocytes for the first donor was assessed.

A control with a CAR encoding an anti-CD19 was added for the *in vivo* experiments.

Mice were grafted intraperitoneally with 1.10⁶ SK-OV-3-Luc tumor cells, then 3 days later, CAR-T cells were injected at a dose of 3.10⁶ per mouse. Tumor growth was monitored for 25 days by IVIS (spectrum imaging system), revealing luciferase from SK-OV-3 cells and correlating with tumor mass size.

Images obtained from mice by PET-Scan was performed on the day of CAR-T injection (D1), then 6 days later (D7), and finally 24 days later (D25) (data not shown).

Similarly, mice injected with PBS or anti-CD19 CAR-T cells saw their tumor mass increase over time. Mice injected with different anti-Her2 CAR-T cells, on the other hand, saw their tumors disappear except for one mouse in the sdAb-armed CAR-T groups.

By combining these two *in vivo* experiments, the inventors can see that sdAb-armed CAR-T cells in both VHH and VH formats are just as effective as scFv-armed CAR-T cells. Knowing that in the experiment, CAR constructs with scFv (anti-CD19 or anti-Her2) were better expressed on the surface of more lymphocytes compared to constructs with anti-Her2 sdAbs (VHH or VH).

This work has allowed the inventors to demonstrate the efficacy of the sdAb of the invention in CAR-T.

The result of the present invention is the first proof of concept for the use of sdAbs *in vivo* in CAR-T format with an anti-tumor effect against the Her2 target.

The invention can also be defined according to any embodiment listed here under.
1. A humanized synthetic single domain antibody (sdAb) directed against HER2, wherein said anti-HER2 sdAb has the following formula FRW1-CDR1-FRW2-CDR2-FRW3-CDR3-FRW4, and wherein the CDRs consists of:
   CDR1 of SEQ ID NO:7; CDR2 of SEQ ID NO:8 and CDR3 of SEQ ID NO:9 or variants thereof.
2. The anti-HER2 sdAb according to embodiment 1, wherein the framework region consists of:
   FRW1 of SEQ ID NO:3; FRW2 of SEQ ID NO:4; FRW3 of SEQ ID NO:5; FRW4 of SEQ ID NO:6;
   or their functional variants with no more than 0, 1, 2 or 3 conservative amino acid substitutions in each of FRW1, FRW2, FRW3 and FRW4.
3. The anti-HER2 sdAb according to embodiment 2 having a sequence set forth set forth in SEQ ID NO:2.
4. The anti-HER2 sdAb according to any one of embodiments 1-3, which is linked directly or indirectly, covalently or non-covalently to a compound of interest selected from a nucleic acid, a polypeptide or a protein, a virus, a toxin and a chemical entity.
5. The anti-HER2 sdAb according to embodiment 4, wherein the antibody is linked directly or indirectly, covalently or non-covalently to a diagnostic compound selected from an enzyme, a fluorophore, a NMR or MRI contrast agent, a radioisotope and a nanoparticle.
6. The anti-HER2 sdAb according to embodiment 4, wherein the antibody is linked directly or indirectly, covalently or non-covalently to a therapeutic compound selected from cytotoxic agents, chemotherapeutic agents, radioisotopes, targeted anti-cancer agents, immunotherapeutic agents (such as immunosuppressants or immune stimulators), and lytic peptides.
7. The anti-HER2 sdAb according to any one of embodiments 1-4, which is linked directly or indirectly, covalently or non-covalently to a drug nanocarrier, optionally an organic nanocarrier.
8. The anti-HER2 sdAb according to any one of embodiments 1-4, wherein the organic nanocarrier is selected from polymeric nanoparticles, liposomes, micelles and protein-based nanocarrier such as albumin, optionally wherein the organic nanocarrier is a liposome.
9. The anti-HER2 sdAb according to any one of embodiment 7 or 8, wherein, the drug encapsulated into the nanocarrier include a therapeutic compound or a diagnostic compound, optionally wherein the therapeutic compound is a cytotoxic compound.
10. The anti-HER2 sdAb according to any one of embodiment 1-4 which is fused to an immunoglobulin domain, optionally, which is fused to an Fc domain.
11. A multispecific binding compound comprising at least a fist sdAb consisting in the anti-HER2 sdAb as defined any one of embodiments 1 to 10, and further comprising another sdAb binding to a second antigen, optionally wherein, the first sdAb is located at the N-terminus of the second sdAb or wherein the first sdAb is located at the C-terminus of the second sdAb.
12. A chimeric antigen receptor (CAR) comprising (a) an antigen binding domain comprising at least a first sdAb consisting in an anti-HER2 sdAb as defined in any one of embodiments 1-4 and optionally a second sdAb specifically binding to a second antigen, (b) a transmembrane domain; and (c) an intracellular domain.
13. The CAR according to embodiment 12, wherein the transmembrane domain is selected from the transmembrane domain of the CD3zeta domain, the CD28 transmembrane domain, the CD8 alpha transmembrane domain, the DAP10 transmembrane domain, or the DAP12 transmembrane domain.
14. The CAR according to any one of embodiments 12 to 13, wherein the intracellular domain comprises one or more domains derived from the CD28, the OX40, the CD3zeta, the 4-1BB, the DAP10 and/or the DAP12 intracellular domains, optionally wherein the intracellular domain comprises the CD3zeta and 4-1BB intracellular domains.
15. The CAR according to any one of embodiments 12-14, wherein the transmembrane domain is the transmembrane domain of CD8 alpha and the intracellular domain comprises the CD3 zeta and 4-1BB intracellular domains.
16. The CAR according to any one of embodiments 12-15, which further comprises a spacer and/or a hinge domain located between the C-terminus domain of the extracellular antigen binding domain and the N-terminus of the transmembrane domain, optionally wherein the hinge is the hinge of CD8 alpha.
17. The CAR according to any one of embodiments 12-16, which further comprises a signal peptide located at the N-terminus of the polypeptide.
18. An isolated nucleic acid comprising a nucleic acid sequence encoding the anti-HER2 sdAb or the CAR according to any one of embodiments 1-17.
19. The isolated nucleic acid according to embodiment 18, wherein the anti-HER2 sdAb, or the CAR is linked to a heterologous regulatory control sequence.
20. A vector comprising the nucleic acid of any one of embodiment 18 or 19.
21. A host cell comprising a nucleic acid according to embodiment 18 or 19 or a vector according to embodiment 20.
22. An isolated cell or population of cells expressing the anti-HER2 sdAb, or the CAR according to any one of embodiments 1-17,
23. An isolated cell or cell population according to embodiment 22, wherein said cell is an allogenic or autologous cell selected from macrophages, NK cells, CD4+/CD8+, TILs/tumor derived CD8 T cells, central memory CD8+ T cells, Treg, MAIT, and Yδ T cells.
24. The anti-HER2 sdAb, the CAR, the nucleic acid, the vector, the host cell, the isolated cell or cell population as defined in any one of embodiments 1-23, for use in therapy
25. The anti-HER2 sdAb, the CAR, the nucleic acid, the vector, the host cell, the isolated cell or cell population as defined in any one of embodiment 1-23, for use in the treatment of cancer in a subject in need thereof.
26. The anti-HER2 sdAb, the CAR, the nucleic acid, the vector, the host cell, the isolated cell or cell population as defined in any one of embodiments 1-23, for use in therapy according to embodiment 24 or 25, wherein said anti-HER2 sdAb, CAR, nucleic acid, vector host cell, isolated cell or cell population is used in combination with at least one further therapeutic agent, wherein said at least one further therapeutic agent is an anticancer agent, optionally a chemotherapeutic agent, or an immunotherapeutic agent, optionally a checkpoint inhibitor.
27. Use of anti-HER2 sdAb according to embodiment 4, 5 or 9 for the detection or monitoring of a HER2-mediated cancer.
28. An *in vitro* or *ex vivo* method for diagnosing or monitoring an HER2 mediated cancer in a subject comprising the steps of:
   a) Contacting *in vitro* an appropriate sample from said subject with the diagnostic agent as defined in embodiment 5 or 9, and
   b) Determining the expression of HER2 in said sample.

### REFERENCES:

Throughout this application, various references describe the state of the art to which this invention pertains. The disclosures of these references are hereby incorporated by reference into the present disclosure.
Blanchard N, Decraene M, Yang K, Miro-Mur F, Amigorena S, Hivroz C. Strong and durable TCR clustering at the T/dendritic cell immune synapse is not required for NFAT activation and IFN-gamma production in human CD4+ T cells. J Immunol. 2004 Sep 1;173(5):3062-72. doi: 10.4049/jimmunol.173.5.3062. PMID: 15322166.
Burstein HJ. The distinctive nature of HER2-positive breast cancers, N Engl J Med. 2005;353:1652-1654.
Ruschoff J et al., HER2 testing in gastric cancer: a practical approach. Mod Pathol. 2012;25:637-650.
Meza-Junco J, Au HJ, Sawyer MB. Critical appraisal of trastuzumab in treatment of advanced stomach cancer, Cancer Manag Res. 2011;3:57-64.
Chiosea SI, et al., Molecular characterization of apocrine salivary duct carcinoma. Am J Surg Pathol. 2015;39:744-752.
Santin AD et al., Trastuzumab treatment in patients with advanced or recurrent endometrial carcinoma overexpressing HER2/neu. Int J Gynecol Obstet. 2008;102:128-131.
Vasconcellos FA et al., Generation and characterization of new HER2 monoclonal antibodies. Acta Histochem. 2013;115:240-244.
Pollock NI, Grandis JR., HER2 as a therapeutic target in head and neck squamous cell carcinoma. Clin Cancer Res. 2015;21:526-533.
Wu X, Chen S, Lin L, et al. A Single Domain-Based Anti-Her2 Antibody Has Potent Antitumor Activities. Transl Oncol. 2018;11(2):366-373.
Y. Guo, Y et al., Chimeric antigen receptor-modified T cells for solid tumors: challenges and prospects, J Immunol Res, 2016; J. Li et al., Chimeric antigen receptor T cell (CAR-T) immunotherapy for solid tumors: lessons learned and strategies for moving forward; J Hematol Oncol, 11 (2018), p. 22.
Xin He, Zijie Feng, Jian Ma, Sunbin Ling, Yan Cao, Buddha Gurung, Yuan Wu, Bryson W. Katona, Kienan P. O'Dwyer, Don L. Siegel, Carl H. June, Xianxin Hua. Bispecific and split CAR T cells targeting CD13 and TIM3 eradicate acute myeloid leukemia. Blood 2020; 135 (10): 713-723. doi: https://doi.org/10.1182/blood.2019002779.
Morgan RA, Yang JC, Kitano M, Dudley ME, Laurencot CM, Rosenberg SA. Case report of a serious adverse event following the administration of T cells transduced with a chimeric antigen receptor recognizing ERBB2. Mol Ther. 2010 Apr;18(4):843-51. doi: 10.1038/mt.2010.24. Epub 2010 Feb 23. PMID: 20179677; PMCID: PMC2862534.
Long AH, Haso WM, Shern JF, Wanhainen KM, Murgai M, Ingaramo M, Smith JP, Walker AJ, Kohler ME, Venkateshwara VR, Kaplan RN, Patterson GH, Fry TJ, Orentas RJ, Mackall CL. 4-1BB costimulation ameliorates T cell exhaustion induced by tonic signaling of chimeric antigen receptors. Nat Med. 2015 Jun;21(6):581-90. doi: 10.1038/nm.3838. Epub 2015 May 4. PMID: 25939063; PMCID: PMC4458184.
Linguanti F, Abenavoli EM, Berti V, Lopci E. Metabolic Imaging in B-Cell Lymphomas during CAR-T Cell Therapy. Cancers (Basel). 2022 Sep 27;14(19):4700. doi: 10.3390/cancers14194700. PMID: 36230629; PMCID: PMC9562671.

## Claims

1. A humanized synthetic single domain antibody (sdAb) directed against HER2, wherein said anti-HER2 sdAb has the following formula FRW1-CDR1-FRW2-CDR2-FRW3-CDR3-FRW4, and wherein the CDRs consists of:
CDR1 of SEQ ID NO:7; CDR2 of SEQ ID NO:8 and CDR3 of SEQ ID NO:9 or variants thereof.

2. The anti-HER2 sdAb according to claim 1, wherein the framework region consists of:
FRW1 of SEQ ID NO:3; FRW2 of SEQ ID NO:4; FRW3 of SEQ ID NO:5; FRW4 of SEQ ID NO:6;
or their functional variants with no more than 0, 1, 2 or 3 conservative amino acid substitutions in each of FRW1, FRW2, FRW3 and FRW4.

3. The anti-HER2 sdAb according to claim 2 having a sequence set forth set forth in SEQ ID NO:2.

4. The anti-HER2 sdAb according to any one of claims 1-3, which is linked directly or indirectly, covalently or non-covalently to a compound of interest selected from a nucleic acid, a polypeptide or a protein, a virus, a toxin and a chemical entity.

5. The anti-HER2 sdAb according to any one of claims 1-4 which is fused to an immunoglobulin domain, optionally, which is fused to an Fc domain.

6. A multispecific binding compound comprising at least a fist sdAb consisting in the anti-HER2 sdAb as defined any one of claims 1 to 5, and further comprising another sdAb binding to a second antigen, optionally wherein, the first sdAb is located at the N-terminus of the second sdAb or wherein the first sdAb is located at the C-terminus of the second sdAb.

7. A chimeric antigen receptor (CAR) comprising (a) an antigen binding domain comprising at least a first sdAb consisting in an anti-HER2 sdAb as defined in any one of claims 1-4 and optionally a second sdAb specifically binding to a second antigen, (b) a transmembrane domain; and (c) an intracellular domain.

8. The CAR according to claim 7, wherein the transmembrane domain is selected from the transmembrane domain of the CD3zeta domain, the CD28 transmembrane domain, the CD8 alpha transmembrane domain, the DAP10 transmembrane domain, or the DAP12 transmembrane domain.

9. The CAR according to any one of claim 7 or 8, wherein the intracellular domain comprises one or more domains derived from the CD28, the OX40, the CD3zeta, the 4-1BB, the DAP10 and/or the DAP12 intracellular domains, optionally wherein the intracellular domain comprises the CD3zeta and 4-1BB intracellular domains.

10. The CAR according to any one of claims 7-9, wherein the transmembrane domain is the transmembrane domain of CD8 alpha and the intracellular domain comprises the CD3 zeta and 4-1BB intracellular domains.

11. The CAR according to any one of claims 7-10, which further comprises a spacer and/or a hinge domain located between the C-terminus domain of the extracellular antigen binding domain and the N-terminus of the transmembrane domain, optionally wherein the hinge is the hinge of CD8 alpha.

12. The CAR according to any one of claims 7-11, which further comprises a signal peptide located at the N-terminus of the polypeptide.

13. An isolated nucleic acid comprising a nucleic acid sequence encoding the anti-HER2 sdAb or the CAR according to any one of claims 1-12, in particular wherein the nucleic acid sequence encoding the anti-HER2 sdAb, or the CAR is linked to a heterologous regulatory control sequence, or a vector comprising a nucleic acid sequence encoding the anti-HER2 sdAb or the CAR according to any one of claims 1-12, in particular wherein the nucleic acid sequence encoding the anti-HER2 sdAb, or the CAR is linked to a heterologous regulatory control sequence.

14. A cell, in particular an isolated cell, a host cell, or a population of cells, expressing the anti-HER2 sdAb or the CAR according to any one of claims 1-12, and/or comprising a nucleic acid or a vector according to claim 13.

15. The anti-HER2 sdAb, the CAR, the nucleic acid, the vector, the host cell, the isolated cell or cell population as defined in any one of claims 1-14, for use in therapy, in particular for use in the treatment of cancer in a subject in need thereof.

16. The anti-HER2 sdAb, the CAR, the nucleic acid, the vector, the host cell, the isolated cell or cell population as defined in any one of claims 1-14, for use in therapy according to claim 15, wherein said anti-HER2 sdAb, CAR, nucleic acid, vector host cell, isolated cell or cell population is used in combination with at least one further therapeutic agent, wherein said at least one further therapeutic agent is an anticancer agent, optionally a chemotherapeutic agent, or an immunotherapeutic agent, optionally a checkpoint inhibitor.

17. Use of anti-HER2 sdAb according to claim 1 to 5, for the detection or monitoring of a HER2-mediated cancer.

18. An *in vitro* or *ex vivo* method for diagnosing or monitoring an HER2 mediated cancer in a subject comprising the steps of:
c) Contacting *in vitro* an appropriate sample from said subject with the diagnostic agent as defined in any one of claims 1 to 5, and
d) Determining the expression of HER2 in said sample.

## Amended claims

### Amended claims in accordance with Rule 137(2) EPC.

1. A humanized synthetic single domain antibody (sdAb) directed against HER2, wherein said anti-HER2 sdAb has the following formula FRW1-CDR1-FRW2-CDR2-FRW3-CDR3-FRW4, and has the sequence set forth in SEQ ID NO: 2.

2. The anti-HER2 sdAb according to claim 1, which is linked directly or indirectly, covalently or non-covalently to a compound of interest selected from a nucleic acid, a polypeptide or a protein, a virus, a toxin and a chemical entity.

3. The anti-HER2 sdAb according to claim 1 or 2 which is fused to an immunoglobulin domain, optionally, which is fused to an Fc domain.

4. A multispecific binding compound comprising at least a first sdAb consisting in the anti-HER2 sdAb as defined in any one of claims 1 to 3, and further comprising another sdAb binding to a second antigen, optionally wherein, the first sdAb is located at the N-terminus of the second sdAb or wherein the first sdAb is located at the C-terminus of the second sdAb.

5. A chimeric antigen receptor (CAR) comprising (a) an antigen binding domain comprising at least a first sdAb consisting in an anti-HER2 sdAb as defined in any one of claims 1 to 3and optionally a second sdAb specifically binding to a second antigen, or a multispecific binding compound as defined in claim 4; (b) a transmembrane domain; and (c) an intracellular domain.

6. The CAR according to claim 5, wherein the transmembrane domain is selected from the transmembrane domain of the CD3zeta domain, the CD28 transmembrane domain, the CD8 alpha transmembrane domain, the DAP10 transmembrane domain, or the DAP12 transmembrane domain.

7. The CAR according to any one of claim 5 or 6, wherein the intracellular domain comprises one or more domains derived from the CD28, the OX40, the CD3zeta, the 4-1BB, the DAP10 and/or the DAP12 intracellular domains, optionally wherein the intracellular domain comprises the CD3zeta and 4-1BB intracellular domains.

8. The CAR according to any one of claims 5 to 7, wherein the transmembrane domain is the transmembrane domain of CD8 alpha and the intracellular domain comprises the CD3 zeta and 4-1BB intracellular domains.

9. The CAR according to any one of claims 5 to 8, which further comprises a spacer and/or a hinge domain located between the C-terminus domain of the extracellular antigen binding domain and the N-terminus of the transmembrane domain, optionally wherein the hinge is the hinge of CD8 alpha.

10. The CAR according to any one of claims 5 to 9, which further comprises a signal peptide located at the N-terminus of the polypeptide.

11. An isolated nucleic acid comprising a nucleic acid sequence encoding the anti-HER2 sdAb or the CAR according to any one of claims 1 to 10, in particular wherein the nucleic acid sequence encoding the anti-HER2 sdAb, or the CAR is linked to a heterologous regulatory control sequence, or a vector comprising a nucleic acid sequence encoding the anti-HER2 sdAb or the CAR according to any one of claims 1 to 10, in particular wherein the nucleic acid sequence encoding the anti-HER2 sdAb, or the CAR is linked to a heterologous regulatory control sequence.

12. A cell, in particular an isolated cell, a host cell, or a population of cells, expressing the anti-HER2 sdAb or the CAR according to any one of claims 1 to 10, and/or comprising a nucleic acid or a vector according to claim 11.

13. The anti-HER2 sdAb, the CAR, the nucleic acid, the vector, the host cell, the isolated cell or cell population as defined in any one of claims 1 to 12, for use in therapy, in particular for use in the treatment of cancer in a subject in need thereof.

14. The anti-HER2 sdAb, the CAR, the nucleic acid, the vector, the host cell, the isolated cell or cell population as defined in any one of claims 1 to 12, for use in therapy according to claim 13, wherein said anti-HER2 sdAb, CAR, nucleic acid, vector host cell, isolated cell or cell population is used in combination with at least one further therapeutic agent, wherein said at least one further therapeutic agent is an anticancer agent, optionally a chemotherapeutic agent, or an immunotherapeutic agent, optionally a checkpoint inhibitor.

15. *In vitro* use of anti-HER2 sdAb according to claim 1 to 3, for the detection or monitoring of a HER2-mediated cancer.

16. An *in vitro* or *ex vivo* method for diagnosing or monitoring an HER2 mediated cancer in a subject comprising the steps of:
a) Contacting *in vitro* an appropriate sample from said subject with the diagnostic agent as defined in any one of claims 1 to 5, and
b) Determining the expression of HER2 in said sample.
